Europäisches Patentamt

European Patent Office

Office européen des brevets

(19)

(11) EP 0 478 502 B1

(12) **EUROPEAN PATENT SPECIFICATION**

(45) Date of publication and mention
of the grant of the patent:
**20.02.2002 Bulletin 2002/08**

(51) Int Cl.[7]: **C12N 9/04**, C12N 15/53,
C12N 9/99, A01H 5/00,
A01N 25/00

(21) Application number: **91810714.5**

(22) Date of filing: **05.09.1991**

(54) **Histidinol dehydrogenase protein, DNA and muteins and transgenic plants thereof**

Histidinol Dehydrogenase Protein, DNS und Muteine und diese enthaltende transgenische Pflanzen

Protéine de déhydrogénase de histidinol, ses ADN et mutéines et plantes transgéniques

(84) Designated Contracting States:
**AT BE CH DE DK ES FR GB GR IT LI LU NL SE**

(30) Priority: **14.09.1990 US 583892**

(43) Date of publication of application:
**01.04.1992 Bulletin 1992/14**

(73) Proprietor: **Syngenta Participations AG**
**4058 Basel (CH)**

(72) Inventors:
• **Scheidegger, Alfred**
**Nishinomiya-shi, 662 (JP)**
• **Ward, Eric R.**
**Durham, NC 27701 (US)**
• **Ryals, John A.**
**Durham, NC 27713 (US)**
• **Nagai-Hayashi, Atsuko**
**Amagasaki-shi, 661 (JP)**

(56) References cited:
**EP-A- 0 290 406          EP-A- 0 360 750**
**EP-A- 0 366 612**

• **PHYTOCHEMISTRY, vol. 20, no. 8, 1981, Oxford
(GB); S. WONG et al., pp. 1831-1834**
• **TRAC, TRENDS IN ANALYTICAL CHEMISTRY,
vol. 2, no. 4, 1983, Cambridge (GB); P. DEAN, pp.
80-83**
• **JOURNAL OF BIOCHEMISTRY, vol. 93, no. 1,
1983, Tokyo (JP); N. SAKIHAMA et al., pp.
129-134**
• **JOURNAL OF BIOLOGICAL CHEMISTRY, vol.
254, no. 15, 1979, Baltimore (US); J.K. KEESEY
et al., pp. 7427-7433**
• **PROCEEDINGS OF THE NATL. ACADEMY OF
SCIENCES USA, vol. 88, May 1991, Washington,
DC (US); A. NAGAI et al., pp. 4133-4137**
• **ARCHIVES OF BIOCHEMISTRY & BIOPHYSICS,
vol. 284, no. 1, 1991, New York, NY (US); A.
NAGAI et al., pp. 127-132**

**Description**

The present invention relates to methods of purifying a previously unpurified protein from plants, the protein purified by such a method, DNA coding for such a protein, methods of using the protein and DNA to assay for specific inhibitors of the protein, and methods of using the specific inhibitors to assay for altered forms of the protein that are less functional forms of the protein so as to produce an insensitivity to the inhibitors.

Histidinol dehydrogenase [L-Histidinol-NAD Oxido-Reductase (EC 1.1.1.23)] catalyzes the final two steps in the biosynthesis of the amino acid histidine. This reaction is an oxidation of histidinol to histidinal to histidine, which is coupled to reduction of two moles of the required cofactor NAD per mole of histidine formed.

$$\text{Histidinol} + \text{NAD}^+ \rightarrow \text{Histidine} + 2 \text{ NADH}$$

While the enzyme and gene encoding it have been well characterized in the bacteria *Salmonella typhimurium* [Yourno and Ino (1968); Gorisch and Holke (1985); and Grubmeyer *et al* (1989)] and *Escherichia coli* [Bitar *et al* (1977)] and the yeast *Saccharomyces cereviseae* [Shaffer *et al* (1972); and Keesey *et al* (1979)], this enzyme has not been previously purified to homogeneity from any plant sources. This is in part because enzymes for primary metabolic functions such as amino acid biosynthesis are especially difficult to purify due to their low concentration in the source tissue. In plants, this difficulty is compounded by the existence of phenolic compounds and other secondary metabolites that can react with proteins throughout their purification.

Histidine biosynthesis in general in higher plants has not been studied well. Some evidence for the existence in plants of a biosynthetic pathway similar to that in microorganisms has been obtained from *in vivo* experiments using various different plants and a blue-green algae [Dougall and Fulton (1967); Negrutiu *et al* (1985); Heim *et al* (1989); Yavada (1979)].

Histidinol dehydrogenase activity has been detected in ten different plant species: asparagus, cabbage, cucumber, egg plant, lettuce, radish [Wong and Mazelis (1981)], rose, squash [Wong and Mazelis (1981)], turnip [Wong and Mazelis (1981)], and wheat [Wong and Mazelis (1981) and this work] and in 5 preparations of distinct cell differentiation: germ [[Wong and Mazelis (1981)] and this work), root [Wong and Mazelis (1981)], fruit [Wong and Mazelis (1981) and this work], shoot, and cultured cell.

Only a few attempts have been made to study the enzymes involved in histidine (His) biosynthesis in higher plants. The hitherto impossible task of purifying to homogeneity any histidine biosynthesis enzyme from plants seems to be the limiting step in biochemical investigations. In crude extracts from shoots of barley, oats and peas, the activities of ATP-phosphoribosyl transferase, imidazoleglycerol phosphate dehydratase and histidinol phosphate phosphatase were detected [Wiater *et al* (1971)].

Histidinol dehydrogenase activity has been found in crude extracts of different plant species [Wong *et al* (1981)]. Partial amino acid sequences of the cabbage enzyme are described by Nagai et al. These data suggest that histidine biosynthesis in plants follows the same pathway as in microorganisms, although no protein has ever been isolated from plants and identified as an enzyme involved directly in histidine biosynthesis.

The pathways for biosynthesis of the ten amino acids essential to the human diet are of special interest as targets for development of novel inhibitory compounds that could act as herbicides. This interest is due to the likelihood that a specific inhibitor of any enzyme from these pathways will be completely non-toxic to vertebrates, which lack the biosynthetic pathways for the essential amino acids.

It was, therefore, one of the main objectives of the present invention to develop a method for purifying the previously unpurified histidinol dehydrogenase enzyme from plants.

It was a further objective to generate DNA sequences coding for a protein exhibiting histidinol dehydrogenase activity using plants as a source organism.

It was also an objective to develop an assay for identifying novel inhibitory compounds that could act as herbicides.

These and other objectives could be surprisingly met within the present invention.

Accordingly, the present invention comprises a plant protein exhibiting histidinol dehydrogenase activity, having an amino acid sequence identical or homologous to at least 70 percent, preferably 80 percent, more preferably 90 percent, and most preferably 99 percent of the amino acid sequence of SEQ ID NO: 1.

The present invention also comprises a method for producing a purified recombinant plant protein exhibiting histidinol dehydrogenase activity comprising:

(a) transforming a suitable host organism with a recombinant DNA comprising a DNA sequence encoding a protein exhibiting histidinol dehydrogenase activity;
(b) isolating the protein from the expression supernatant.

The invention also comprises a method of producing an essentially pure DNA sequence coding for a protein exhibiting histidinol dehydrogenase activity, which method comprises:

(a) preparing a genomic or a cDNA library from a suitable source organism using an appropriate cloning vector;
(b) hybridizing the library with a probe molecule;
(c) identifying positive hybridizations of the probe to the DNA clones from the library that is clones potentially containing the nucleotide sequence corresponding to the amino acid sequence for histidinol dehydrogenase.

Also comprised is a method of producing an essentially pure DNA sequence coding for a protein exhibiting histidinol dehydrogenase activity according to the invention, which method comprises:

(a) preparing total DNA from a genomic or a cDNA library;
(b) using the DNA of step (a) as a template for PCR reaction with primers representing low degeneracy portions of the amino acid sequence of histidinol dehydrogenase.

The invention also comprises a DNA sequence coding for a plant protein exhibiting histidinol dehydrogenase activity identical of homologous to at least 60 percent, preferably 75 percent, more preferably 90 percent of the DNA sequence of SEQ ID NO: 1.

Another aspect of the present invention is an assay to identify inhibitors of a protein exhibiting histidinol dehydrogenase activity comprising:

(a) incubating a first sample of a protein exhibiting a histidinol dehydrogenase activity according to the invention and its substrate;
(b) measuring an uninhibited reactivity of the histidinol dehydrogenase from step (a);
(c) incubating a first sample of histidinol dehydrogenase and its substrate in the presence of a second sample comprising an inhibitor compound;
(d) measuring an inhibited reactivity of the histidinol dehydrogenase from step (c); and
(e) comparing the inhibited reactivity to the uninhibited reactivity of the histidinol dehydrogenase.

Another aspect of the present invention is an assay to identify inhibitor-resistant mutants of a protein exhibiting histidinol dehydrogenase activity comprising:

(a) incubating a first sample of a protein exhibiting a histidinol dehydrogenase activity according to the invention and its substrate in the presence of a second sample comprising a histidinol dehydrogenase inhibitor,
(b) measuring an unmutated reactivity of the histidinol dehydrogenase from step (a);
(c) incubating a first sample of a mutated histidinol dehydrogenase and its substrate in the presence of a second sample comprising a histidinol dehydrogenase inhibitor;
(d) measuring a mutated reactivity of the mutated histidinol dehydrogenase from step (c); and
(e) comparing the mutated reactivity to the unmutated reactivity of the histidinol dehydrogenase.

To assist in interpreting the meaning and scope of the present invention, the following terms are intended to have the meanings as described below, unless otherwise indicated. All references cited in this application are hereby incorporated by reference for their relevant teachings.

Coding DNA Sequence: A DNA sequence which, when transcribed and translated, results in the formation of a cellular polypeptide.

Gene: A discrete chromosomal region comprising regulatory DNA sequences responsible for the control of expression, i.e., transcription and translation, and of a coding sequence which is transcribed and translated to give a distinct polypeptide.

Histidinol Dehydrogenase Inhibitory Effective Amount or Significant Decrease in Histidinol Dehydrogenase Activity: A significant decrease in the ability of histidinol dehydrogenase to convert substrates such as histidinol into products such as histidine as measured by quantifying enzymatic activity as generally known to those of skill in the art, including equilibrium constants, reaction velocities of the appearance of reaction products or the consumption of reaction substrates, reaction kinetics, thermodynamics of reaction, spectrophotometric analysis of reaction products, detection of labelled reaction components, etc, with the preferred method measurement being the spectrophotometric measurement of NAD+ as a reaction byproduct. A significant decrease in enzyme activity is any decrease that is larger than the margin of error inherent in the measurement technique, preferably a decrease by 50 %, more preferably a decrease by 90%, more preferably a decrease by 99%, and most preferably a decrease to essentially undetectable levels of enzyme activity.

Substantial Sequence Homology: Substantial functional and/or structural equivalence between sequences of nucleotides or amino acids. Functional and/or structural differences between sequences having substantial sequence homology will be *de minimus*. The sequences that differ from the natural sequences are usually variants of the natural sequence. A variant of a natural sequence is a modified form of the natural sequence that performs the same function. The variant may be a mutation, or may be a synthetic sequence. A *de minimus* functional difference results from a nucleotide or amino acid sequence that codes for a protein having essentially the same characteristics as the native protein. Such characteristics can include, for example, immunological reactivity, enzyme activity, structural protein integrity, etc. Structural differences are considered *de minimus* if there is a significant amount of sequence overlap or similarity between two or more different sequences or if the different sequences exhibit similar physical properties. In the case of a nucleotide sequence, the different sequences will preferably have at least 60 %, more preferably 75 %, most preferably 90 % or more sequence similarity between them. In the case of amino acid sequences, the different sequences have at least 70 %, more preferably 80 %, more preferably 90 %, and most preferably at least 99 % or more similarity between the polypeptides coded for by the amino acid sequences. Physical properties that may be similar include, for example, electrophoretic mobility, chromatography similarities, sediment gradient coefficients, spectrophotometric properties, etc.

Transgenic Plant: A plant having stably incorporated exogenous DNA in its genetic material. Various different methods of introducing the exogenous DNA into plant protoplasts, plant cells or other plant material are known in the art and can be used to produce the transgenic protoplasts, transgenic cells or transgenic plants of the present invention.

Weeds: Any undesired species of plant growing among a culture of cultivated plants such as crops, preferably any individual of a species that is heterogeneous to the species of a homogenous population of plants.

The present invention relates to methods of purifying a previously unpurified protein from plants, the protein purified by such a method, DNA coding for such a protein, methods of using the protein and DNA to assay for specific inhibitors of the protein, and methods of using the specific inhibitors to assay for altered forms of the protein that are less functional forms of the protein so as to produce an insensitivity to the inhibitors.

The method of purification utilizes an affinity chromatography column that is specific for histidinol dehydrogenase. Various known methods can be employed previous to and subsequent to the affinity chromatography to produce a crude extract to apply to the affinity column and to characterize and store the protein isolated by the affinity column.

Such previous steps can include homogenization, filtration, centrifugation, ammonium salt precipitation, desalting, ion exchange chromatography, sonication, etc. In a preferred embodiment of the present invention, the plant to plant tissue, from which the histidinol dehydrogenase is to obtained, is homogenized using, for example, a blender. The homogenate is then preferably passed through course filtration such as a filtration cloth. The filtrate is then preferably adequately centrifuged, preferably at about 30,000 x g for at least 20 minutes. The supernatant is then preferably precipitated using ammonium sulfate at from about 40 % to 70 % saturation, more preferably at about 45 % to 60 % saturation, and the precipitate is collected and desalted using an appropriate desalting technique such as dialysis or gel filtration, preferably using gel filtration with G-25 Sephadex with about 50 mM TRIS/HCl at pH from about 7 to 7.5, preferably pH 7.4.

The desalted protein extract can then be further prepared for affinity chromatography using an appropriate ion exchange column. A preferred ion exchange column is, for example, a Diethyl-amino-ethyl (DEAE) derivatized resin, such as DEAE-cellulose, more preferably DEAE-Toyopearl [a ion-exchange chromatography resin which can be obtained from TOSOH, Ltd. (Tokyo)], equilibrated with a suitable buffer such as the buffer used in the desalting step, most preferably 50 mM TRIS/HCl at about pH 7.4. Elution from the ion exchange column can be accomplished using a linear gradient containing the equilibration buffer and a suitable salt gradient. A preferred salt gradient is a NaCl gradient ranging from 0 mM to at least 500 mM. The eluate from the ion exchange column is then an appropriate crude extract for application to the affinity chromatography column.

Appropriate ligands for the chromatography column are any substrate specific for the enzyme, histidinol dehydrogenase, that is to be isolated. Attachment of the ligand to the column resin will preferably occur at a position on the ligand that will not significantly interfere with the binding of the ligand to the enzyme to be isolated. Preferred ligands for histidinol dehydrogenase are therefore histidinol and histidinal, more preferably histidinol. Any suitable resin may be used to attach the ligand, with a preferred resin being Sepharose-4B. Methods of attaching ligands to chromatography resins are generally known in the art.

A preferred method of preparing a suitable affinity column resin is as follows. Sepharose-4B is activated with epichlorohydrin as previously described [Matsumoto *et al* (1979)]. Epoxyactivated Sepharose-4B gel is suspended in 0.1N NaOH containing 0.5g glucose per g of gel and incubated at 40°C on a shaker for 24h [Kanamori *et al* (1986)]. After washing extensively with water, the gel is suspended in pre-chilled sodium metaperiodate and the suspension shaken for 1h at 4°C. The gel is washed with water, suspended in 0.1 M HCl and incubated at room temperature for 1h. Again, the gel is washed extensively with water, followed by thorough washing with 10 mM sodium phosphate buffer, pH 7.0 at room temperature.

The formyl carrier gel is suspended in a phosphate buffer, preferably a 10 mM sodium phosphate buffer, pH 7.0 containing 100 mM of histidinol dihydrochloride, which is previously neutralized, preferably by adding NaOH, and the suspension incubated at 4°C overnight. Five milligrams of sodium cyanoborohydride per g of gel is then added to the suspension and incubated with shaking at room temperature for 6h. The gel is extensively washed with distilled water and treated with 5 mg sodium borohydride per g of gel for 3h at 4°C to convert the remaining formyl groups into hydroxymethyl groups.

After preparation of a suitable affinity chromatography column resin, the resin is placed in a suitable column, preferably a column of about 2.5 x 8 cm. The crude extract of protein is placed on the affinity column and preferably extensively washed with a suitable buffer, preferably containing an adequate ionic strength of salt, e.g., 10 mM TRIS/HCl buffer, about pH 7.3, containing about 100 mM to 200 mM NaCl, preferably about 140 mM NaCl. The bound enzyme can be eluted using a suitable displacing solvent. A suitable displacing solvent is one containing a substance that will have an affinity to the enzyme to be isolated that is similar to the ligand used in the affinity column. When the enzyme is histidinol dehydrogenase and the ligand is histidinol, a preferred displacing solvent, is one containing imidazole, more preferably one containing about 50 mM TRIS/HCl, pH about 7.3, and imidazole.

Subsequent to the affinity chromatography methods can be employed to characterize and store the protein isolated by the affinity column. These include desalting the eluate from the affinity column into a suitable buffer to remove the displacing solvent. The desalted protein can then be further characterized or concentrated and stored cryogenically, preferably below -50°C, more preferably at about -80°C.

The protein can be characterized using standard techniques such as gel filtration (molecular mass), polyacrylamide gel electrophoresis [Laemmli (1970)] (purity and molecular weight), isoelectric focusing (isoelectric point), amino acid sequencing, appropriate enzyme assays (to measure enzymatic activity), etc.

The enzyme activity is preferably assayed spectrophotometrically by measuring the increase in absorbence at 340 nm due to the reduction of $NAD^+$ in a spectrophotometer such as, for example, a Hitachi U-3120. In a preferred embodiment of the invention the reaction mixture contains 150 mM Gly/NaOH buffer, pH 9.2, 0.5 mM, $MnCl_2$, 2 mM $NAD^+$, 5 mM histidinol, and 2-5 mU of enzyme sample in total volume of 0.5 ml. The reaction is started with the addition of histidinol, and incubated preferably at 30°C. A reference sample containing water instead of histidinol is always run. One unit of enzyme catalyzes the formation of 25 M of NADH per min under the assay conditions.

The activity can also be followed by measuring the formation of His with a amino acid analyzer such as, for example, a Hitachi L-850. The enzyme reaction can be terminated by addition of formic acid (final concentration, 30%) to the reaction mixture. The sample is then evaporated, and the precipitate dissolved in 0.2 N HCl. This preparation is used for His analysis, amino acid sequencing, enzymatic activity, etc.

Determination of amino acid sequencing can be accomplished by known methods, e.g., manual or automated Edman degradation reaction using commercially available instrumentation (Applied Biosystems, Foster City, California). Amino acid sequencing can be performed on the entire full length protein, or sequencing can be performed on fragments or portions of the protein. Such fragments include N-terminal sequencing, sequencing after digestion with Lys-C (Wako Pure Chemical Co., Osaka, Japan), sequencing after digestion with cyanogen-bromide, etc.

Cyanogen bromide cleavage can be carried out *in situ* according to Simpson and Nice (1984). Digestion with pyroglutamate aminopeptidase [Boehringer Mannheim] can be carried out according to Allen (1981).

In the course of LysC digestion protein is preferably digested with endoproteinase Lys-C [Boehringer Mannheim] in 0.1 M tris-HCl (pH 8.5) over a period of 24 hours at room temperature, using an enzyme:substrate ratio of 1:10. The resulting peptides are isolated by means of HPLC [for example, Aquapore C-8 column (1 x 22 cm, Brownlee)]. A linear acetonitrile:isopropanol (1:1) gradient (0 % to 60 %) in 0.1 % TFA can be used as eluant.

Using the amino acid sequence(s), portions of the amino acid sequence that display minimal degeneration of the nucleotide sequences coding for the protein can be used to synthesize oligonucleotide probes. The oligonucleotide probes can be synthesized by phosphoramidite chemistry, e.g., as performed by an Applied Biosystems 380B DNA synthesizer [Foster City, California] using reagents available from the manufacturer.

Examples of the useful portions of the amino acid sequence obtained from cabbage that can be used to synthesize oligonucleotide probes include:

| Lys-C #17 | Thr | Glu | Leu | Ser | Phe | Ala | Lys | | | | |
| Lys-C #18 | Thr | Val | Val | Leu | Ala | Thr | Pro | Pro | Thr | Lys | |
| Lys-C #32 | Glu | Ala | Phe | Asp | Val | Ala | Tyr | Asp | Asn | Ile | Tyr | Ala |
| | Phe | His | Leu | Ala | Gln | Lys | | | | | |
| Lys-C #45 | Lys | Phe | Met | Thr | Val | Gln | Ser | Leu | Thr | Glu | Glu | Gly |
| | Leu | Arg | Asn | Leu | Gly | Pro | Tyr | Val | Ala | Thr | Met | Ala |
| | Glu | Ile | Glu | Gly | Leu | Asp | Ala | | | | |
| Lys-C #48 | Ala | Leu | Ser | His | Ser | Phe | Thr | Val | Phe | Ala | Arg | Asp |
| | Met | Ile | Glu | Ala | Ile | Thr | Phe | Ser | Asn | Leu | Tyr | Ala |
| | Pro | Glu | Lys | | | | | | | | |
| CNBr # 6 | Met | Ala | Glu | Ile | Glu | Gly | Leu | Asp | Ala | His | Lys | Arg |
| | Ala | Val | Thr | Leu | Arg | ? | Lys | Asp | Ile | Glu | |
| CNBr #18 | Leu | Ala | Ile | Pro | Ala | Gln | Ile | Ala | Gly | Arg | Lys | Thr |
| | Val | Val | Leu | Ala | Thr | Pro | | | | | |
| N-terminal | Met | Lys | Ile | Tyr | Arg | Leu | Ser | Glu | Leu | Ser | Phe | ? |
| | Gln | Val | Glu | Asn | Leu | Lys | Ala | Arg | ? | ? | Ile | Asp |

wherein the amino acid terminology is the standard abbreviations.

After labelling with a suitable label such as P-32, these oligonucleotide probes can be used to probe a DNA library, preferably a cDNA library of the mRNA from the organism from which the histidinol dehydrogenase is obtained. Such a library (a cDNA library) of poly A(+) RNA from the source organism can be constructed in an appropriate cloning vector, preferably a cloning vector such as lambdaZAPII (available from Stratagene, La Jolla, California using the Uni-ZAPXR cDNA kit, also available from Stratagene) utilizing methods that are known in the art. Positive hybridization of the oligonucleotide probes to a cDNA clone from the library identifies that clone as potentially containing the nucleotide sequence corresponding to the amino acid sequence for histidinol dehydrogenase.

Alternatively, total DNA from the DNA library, preferably from the cDNA library can be prepared and used as a template for a PCR reaction with primers representing low degeneracy portions of the amino acid sequence. Preferably, the primers used will generate PCR products that represent a significant portion of the nucleotide sequence. Preferred primers that can be used to probe total DNA from the cDNA library include:

> EW25: 5' TTAAGAATTCTAYGAYAAYATHTAYGC 3'
>
> JR03: 5' CCYTCDATYTCNGCCAT 3'

where D indicates any the bases A, T, or G; H indicates the bases A, T, or C; R indicates the purines A or G; Y indicates the pyrimidines T or C. The PCR products can be further probed to determine if they correspond to a portion of the histidinol dehydrogenase gene using a synthetic oligonucleotide probe corresponding to an amino acid fragment sequence located in the interior or middle region of the histidinol dehydrogenase protein. An example of such a probe would include the $^{32}$P labelled probe represented by the formula:

> JR02: 5' ATNGCYTCDATCATRTC 3'

where D indicates any the bases A, T, or G; H indicates the bases A, T, or C; R indicates the purines A or G; Y indicates the pyrimidines T or C.

The PCR product can thus be used, for example, to select and isolate further DNA clones using standard techniques [Maniatis *et al* (1982)]. Nucleotide sequencing of the full length DNA coding for histidinol dehydrogenase can then be obtained using standard techniques [Maxam and Gilbert (1977); and Sanger (1977)] or using commercially available nucleotide sequencing instrumentation [available from Applied Biosystems, Foster City, California and Dupont, Wilmington, Delaware].

The cDNA clones and PCR products prepared as described above or fragments thereof may be used as a hybridization probe in a process of identifying further DNA sequences from a homolgous or a heterologous source organism encoding a protein product that exhibits histidinol dehydrogenase activity. They may also be used as a RFLP marker to determine, for example, the location of the histidinol dehydrogenase gene or of a closely linked trait in the plant genome or for marker assisted breeding [EP-A 306,139; WO 89/07647].

In a preferred embodiment of the present invention a cabbage cDNA, the sequence of which is shown in SEQ ID NO: 1, was used as a hybridization probe under low-stringency conditions to identify two at least partial cDNA clones [designated SA4 and SA5] from wheat encoding a polypeptide product exhibiting histidinol dehydrogenase activity.

Once having identified and isolated the DNA encoding a polypeptide product exhibiting histidinol dehydrogenase activity, a purified protein exhibiting histidinol dehydrogenase activity can be obtained from transgenic expression of the said DNA, i.e., placing a recombinant DNA comprising a DNA sequence coding for a protein exhibiting histidinol dehydrogenase activity into an appropriate bacterial, yeast or other cell expression system.

Suitable hosts include bacteria such as *E. coli* and yeast, including the strain *Saccharomyces cerevisiae*. Other suitable expression system hosts include insect cells grown in culture. These insect cells may be infected with a baculovirus containing a recombinant DNA molecule according to the invention.

Alternatively, the baculovirus may be used to infect the cells of a living insect, and the insect cells used as the expression system host. The expression system host is then allowed to produce an expression supernatant. This allows facile generation of large amounts of purified recombinant histidinol dehydrogenase by isolating the enzyme from the expression supernatant.

Histidinol dehydrogenase from plants has been found to be encoded as a proenzyme. In case of the histidinol dehydrogenase from cabbage this proenzyme comprises a 31-residue amino-terminal peptide not present in the mature enzyme. The amino-terminal extension of the cabbage proenzyme has a sequence composition typical of chloroplast transit peptides.

The amino-terminal extensions of a histidinol dehydrogenase proenzyme from plants having been operably linked to a homologous or heterologous DNA sequence may thus be used for targeting the said DNA sequences into the chloroplast compartment of the plant cell, as described, for example, in EP-A 189,707.

The purified histidinol dehydrogenase can then be used in an assay to identify inhibitors of a protein exhibiting a histidinol dehydrogenase activity, which assay comprises:

(a) incubating a first sample of a protein exhibiting a histidinol dehydrogenase activity and its substrate;
(b) measuring an uninhibited reactivity of the histidinol dehydrogenase from step (a);
(c) incubating a first sample of histidinol dehydrogenase and its substrate in the presence of a second sample comprising an inhibitor compound;
(d) measuring an inhibited reactivity of the histidinol dehydrogenase from step (c); and
(e) comparing the inhibited reactivity to the uninhibited reactivity of the histidinol dehydrogenase.

Suitable histidinol dehydrogenase for the above assay can be obtained using the purification methods of the present invention. Suitable substrates are histidinol or structural analogs that are capable of being converted into an analog of histidine by the histidinol dehydrogenase enzyme, with the preferred substrate being histidinol. Preferably, the substrate is at a concentration of about 1 mM to about 100 mM, more preferably at about 1 mM to about 10 mM, most preferably at about 5 mM.

In addition to the histidinol dehydrogenase enzyme and a suitable substrate, the reaction mixture can contain a suitable buffer, suitable cofactors and suitable divalent cations as a cofactor. A suitable buffer includes any suitable biological buffer that can provide buffering capability at a pH conductive to the reaction requirements of the enzyme. Preferably, the buffer provides buffering capability in the pH range 7.5 to 10.0, more preferably in the pH range of about 9.1 to about 10.0, most preferably at a pH of about 9.2, e.g., 150 mM Gly/NaOH (glycine/sodium hydroxide) at pH 9.2. A preferred cofactor for the histidinol dehydrogenase enzyme is NAD, preferably at a concentration of about 1 mM to about 100 mM, more preferably at about 2 mM. Preferably, the divalent cation is a divalent metal cation, more preferably manganese. The reaction is carried out at a suitable temperature to allow the reaction to proceed. Such a suitable temperature is about 4°C to about 40°C, more preferably from about room temperature to about 35°C, most preferably at about 30°C. The most preferred reaction mixture contains 150 mM Gly/NaOH buffer, pH 9.2, 0.5 mM $MnCl_2$, 2mM NAD+, 5 mM histidinol, and 2-5 mU of enzyme sample in total volume of 0.5 ml at 30°C. Preferably, the reaction is started with the addition of histidinol.

Uninhibited reactivity of the histidinol dehydrogenase is any measure of enzymatic activity of the histidinol dehydrogenase enzyme while in the presence of a suitable substrate. Such measures of enzymatic activity are generally known to those of skill in the art, including equilibrium constants, reaction velocities of the appearance of reaction products or the consumption of reaction substrates, reaction kinetics, thermodynamics of reaction, spectrophotometric analysis of reaction products, detection of labelled reaction components, etc. See, generally, Segel, Biochemical Calculations, 2nd Edition, John Wiley and Sons, New York (1976); Suelter, A Practical Guide to Enzymology, John Wiley and Sons, New York (1985). The preferred method of measuring enzymatic activity is spectophotometrically by measuring the increase in absorbence at 340nm due to the resuction of NAD$^+$, e.g., using, for example, a Hitachi U-3120 spectrophotometer.

Suitable inhibitor compounds are identified using the above methods. To date at least seven compounds have been so identified.

Inhibited reactivity is determined in the same manner as uninhibited reactivity with the addition of an inhibitor of histidinol dehydrogenase. The concentration of the inhibitor may vary depending on the inhibitory activity, but generally it will be in an amount ranging from about 10 nM to about 200 mM, more preferably about 0.1 mM to about 100 mM, more preferably about 1 mM to about 10 mM. Generally, the histidinol or other substrate will be added to a mixture containing the enzyme and inhibitor and then an enzyme activity determined as described previously.

Comparing the inhibited reactivity to the uninhibited reactivity of the histidinol dehydrogenase includes determining whether a significant decrease in enzyme activity is observed in the inhibited reactivity compared to the uninhibited reactivity. A significant decrease is a decrease in enzymatic activity that is larger than the margin of error inherent in the measurement technique, preferably a decrease by about 50% of the activity in the absence of the inhibitor, more preferably a decrease by about 90%, more preferably a decrease by about 99%, most preferably a decrease to a level of enzymatic activity that is essentially undetectable.

Once an herbicidal compound is identified that inhibits function of the wild-type enzyme, thus killing the organism containing the enzyme, resistant mutants can be isolated by mutagenizing a population of the organism in question, growing the mutagenized population in the presence of a concentration of the inhibitor sufficient to inhibit growth of the wild-type organism, and selecting individuals from the population that are able to grow more rapidly than wild-type organisms.

Mutagenesis in vivo can be by any of several means, including chemical, e.g., ethyl methanesulfonate [Miller, Experiments in Molecular Genetics, Cold Spring Harbor Laboratory, Cold Spring Harbor, New York (1972); Davis et al, Advanced Bacterial Genetics, Cold Spring Harbor Laboratory, Cold Spring Harbor, New York (1980); Sherman et al, Methods in Yeast Genetics, Cold Spring Harbor Laboratory, Cold Spring Harbor, New York (1983); and Somerville and Ogren, In Edelman et al (eds) Methods in Chloroplast Molecular Biology, Elsevier Biomedical Press, pp. 129-138 (1982)], ultraviolet mutagenesis [Miller (1972)], X-ray mutagenesis [e.g., Hake and Freeling, (1986); and Poethig, (1988)] and gamma irradiation. Alternatively, the sequence encoding histidinol dehydrogenase can be mutagenized in vitro by any of several chemical or enzymatic treatments [e.g., Zoller and Smith, (1983); Leung et al, (1989); and Shortle and Bolstein, (1983)]. Such mutated sequences are re-introduced into the host organism, preferably a microbe, and the population containing a mixture of reintroduced mutated sequences is grown on a toxic concentration of the inhibitor. Individuals are directly selected for their ability to grow in the presence of this toxic amount of inhibitor, which ability is conferred by the introduced mutated sequence.

The mutant individuals that result from in vivo mutagenesis, having an ability to tolerate normally toxic concentrations of the inhibitor, can be identified and genetically purified (either by streaking to single colonies several times for the case of a microbe, or by repeatedly selecting a single seed, growing to maturity, and self-pollinating for the case of a plant), the gene encoding histidinol dehydrogenase can be rapidly isolated from the mutant by the polymerase chain reaction and its DNA sequence determined and translated into a predicted amino acid sequence. Amino acids found in the mutant to differ from wild type can be assumed to be responsible for the inhibitor-resistant phenotype. Preferably, the causal nature of the amino acid changes can be directly tested by expressing the mutated sequence in a microbial or plant system, and demonstrating that introduction of the mutated sequence is sufficient to confer an inhibitor-resistant phenotype on an otherwise wild-type individual.

An assay to identify inhibitor-resistant mutant of a protein exhibiting histidinol dehydrogenase activity comprises:

(a) incubating a first sample of a protein exhibiting a histidinol dehydrogenase activity and its substrate in the presence of a second sample comprising a histidinol dehydrogenase inhibitor,
(b) measuring an unmutated reactivity of the histidinol dehydrogenase from step (a);
(c) incubating a first sample of a mutated histidinol dehydrogenase and its substrate in the presence of a second sample comprising a histidinol dehydrogenase inhibitor,
(d) measuring a mutated reactivity of the mutated histidinol dehydrogenase from step (c); and
(e) comparing the mutated reactivity to the unmutated reactivity of the histidinol dehydrogenase.

The reaction mixture and the reaction conditions are the same as for the assay to identify inhibitors of histidinol dehydrogenase (inhibitor assay) with the following modifications. First, a histidinol dehydrogenase mutant, obtained as described above, is substituted in one of the reaction mixtures for the wild-type histidinol dehydrogenase of the inhibitor assay. Second, an inhibitor of wild-type histidinol dehydrogenase is present in both reaction mixtures. Third, mutated reactivity (enzyme activity in the presence of inhibitor and mutated histidinol dehydrogenase) and unmutated reactivity (enzyme activity in the presence of inhibitor and wild-type histidinol dehydrogenase) are compared to determine whether a significant increase in enzyme activity is observed in the mutated reactivity when compared to the unmutated reactivity. Mutated reactivity is any measure of enzymatic activity of the mutated histidinol dehydrogenase enzyme while in the presence of a suitable substrate and the inhibitor. Unmutated reactivity is any measure of enzymatic activity of the wild-type histidinol dehydrogenase enzyme while in the presence of a suitable substrate and the inhibitor. A significant increase is a increase in enzymatic activity that is larger than the margin of error inherent in the measurement technique, preferably an increase by about 2-fold of the activity of the wild-type enzyme in the presence of the inhibitor, more preferably an increase by about 10-fold, most preferably an increase by about 100-fold.

A mutated histidinol dehydrogenase-encoding DNA sequence that confers herbicide resistance can then be introduced into a crop species of interest, allowing the crop to survive in the presence of a concentration of the herbicide that kills all plants lacking the resistant form of the enzyme.

To become expressed in the plant cell the mutated histidinol dehydrogenase-encoding DNA sequence is preferably cloned into a suitable vector for plant transforamtion which preferably comprise one or more promoter or regulatory DNA sequences to promote expression of the coding DNA sequence in the transformed plant cell.

Suitable control sequences are those comprising promoter and 5' and 3' untranslated regulatory sequences that are functional in plants. These sequences may, independently, be derived from any source, such as, for example, virus, plant or bacterial genes. These promoters or regulatory sequences can be constitutive in nature or can be regulated in their patterns of expression. Such regulation may be temporal or spatial and include developmentaly regulated promoters and inducible promoters. Preferred promoters include promoters from the cauliflower mosaic virus, including the 35S and 19S promoters. Cauliflower mosaic virus (CaMV) has been characterized and described by Hohn *et al* in Current Topics in Microbiology and Immunology, 96:194-220 and appendices A to G (1982). This description is incorporated herein by reference. Also preferred are the nopaline synthase, octopine synthase and mannopine synthase promoters.

The virus promoters and 5' and 3' untranslated sequences suitable for use are functional in plants and include, for example, plant viruses such as cauliflower mosaic virus.

CaMV is an unusual plant virus in that it contains double-stranded DNA. At least two CaMV transcriptional promoters are functional in plants, namely the 19S promoter, which results in transcription of gene VI of CaMV, and the 35S promoter. The 19S promoter and the 35S promoter are the preferred plant virus promoters for use in the present invention.

CaMV 19S promoters and 5' untranslated regions may be obtained by means of a restriction map such as the map described in Figure 4 on page 199 of the Hohn *et al* article mentioned above, or from the sequence that appears in Appendix C of the Hohn *et al* article.

In order to isolate the CaMV 19S promoter and, optionally, the adjacent 5' untranslated region, a restriction fragment of the CaMV genome containing the desired sequences is selected. A suitable restriction fragment that contains the 19S promoter and the 5' untranslated region is the fragment between the PstI site starting at position 5386 and the HindIII site starting at position 5850 of Figure 4 and appendix C of the Hohn *et al*, article.

Undesired nucleotides in the restriction fragment may optionally be removed by standard methods. Some suitable methods for deleting undesired nucleotides include the use of exonucleases [Maniatis *et al* (1982); pages 135-139] PCR technology [Mullis *et al* (1987); Erlich, (ed.), PCR Technology, Stockton Press New York (1989)] and oligonucleotide-directed mutagenesis [Zoller and Smith (1983)].

A similar procedure may be used to obtain a desirable 3' untranslated region. For example, a suitable CaMV gene 3' untranslated sequence may be obtained by isolating the region between the EcoRV site at position 7342 and the BglII site at position 7643 of the CaMV genome as described in Figure 4 and appendix C of the Hohn *et al* article.

Examples of plant gene promoters and 5' and 3' untranslated regions suitable for use in the present invention also include those of the gene coding for the small subunit of ribulose bisphosphate carboxylase oxygenase, phosphoenol pyruvatecarboxylase and chlorophyll a/b-binding protein. These plant gene regions may be isolated from plant cells in ways comparable to those described above for isolating the corresponding regions from CaMV; see Morelli *et al* (1985).

Suitable promoters and 5' and 3' untranslated regions from bacterial genes include those present in the T-DNA region of *Agrobacterium* plasmids. Some examples of suitable Agrobacterium plasmids include the Ti plasmid of *A. tumefaciens* and the Ri plasmid of *A. rhizogenes.* The *Agrobacterium* promoters and 5' and 3' untranslated regions useful in the present invention are, in particular, those present in the genes coding for octopine, mannopine and nopaline synthases. These sequences may be obtained by methods similar to those described above for isolating CaMV and plant promoters and untranslated sequences; see Bevan *et al* (1983).

In addition to a promoter, the chimeric genes of the present invention may preferably include further untranslated sequences at the 5' end, termed a leader sequence. Suitable leader sequences include leader sequences of various lengths isolated from the 35S CaMV gene. Pierce *et al*, Plant Gene Systems and their Biology, (Alan R. Liss, Inc.) pp. 301-310. The preferred leader sequences are those isolated from the 35S CaMV gene, having a length from about 50 to about 130 nucleotides.

The recombinant DNA comprising the mutated histidinol dehydrogenase-encoding DNA sequence can be introduced into the plant cell in a number of ways that are well known to those of skill in the art. For example, methods of transforming plant cells include microinjection [Crossway *et al* (1986); Neuhaus (1987)], electroporation [Riggs *et al* (1986)], *Agrobacterium* mediated transformation (Hinchee *et al* (1988)], direct gene transfer (Paszkowski *et al*, (1984)], and ballistic particle acceleration using, for example, devices available from Agracetus, Inc., Madison, Wisconsin and Dupont, Inc., Wilmington, Delaware [see, for example, Sanford *et al*, U.S. Patent 4,945,050; and McCabe *et al*, (1988)]. Also see, Weissinger *et al* (1988); Sanford *et al*, (1987)(onion); Christou *et al* (1988)(soybean); McCabe *et al* (1988)(soybean); Datta *et al* (1990)(rice); Klein *et al* (1988)(maize); Klein *et al* (1988)(maize); Klein *et al*, (1989) (maize); Fromm *et al* (1990); Gordon-Kamm *et al* (1990) (maize)].

One possible method for introducing genetic material into plant cells comprises, for example, bringing plant cells into contact with viruses or with *Agrobacterium* comprising the DNA to be introduced. This may be achieved by infecting sensitive plant cells or by co-cultivating protoplasts derived from plant cells. Within the scope of this invention, Cauliflower Mosaic Virus (CaMV) may be used as a vector for the insertion of the mutated histidinol dehydrogenase-encoding DNA sequence according to the invention into a plant.

Another method of inserting the mutated histidinol dehydrogenase-encoding DNA sequence into a cell makes use of the infection of the plant cell with *Agrobacterium tumefaciens* and/or *Agrobacterium rhizogenes,* which has previously been transformed with the said gene construction. The transgenic plant cells are then cultured under suitable culture conditions known to the person skilled in the art, so that they form shoots and roots and whole plants are finally formed.

A further possible method of transforming plant material comprises mixed infection using both *Agrobacterium rhizogenes* and transformed *Agrobacterium tumefaciens*, as described by Petit *et al* (1986) for the transformation of carrots.

The mutated histidinol dehydrogenase-encoding DNA sequence according to the invention can therefore be transferred into suitable plant cells by means of, for example, the Ti-plasmid of *Agrobacterium tumefaciens* or the Ri-plasmid of *Agrobacterium rhizogenes*. The Ti-plasmid or Ri-plasmid is transferred to the plant in the course of infection by *Agrobacterium* and integrated in stable manner into the plant genome.

Both Ti-plasmids and Ri-plasmids have two regions that are essential for the production of transformed cells. One of those regions, the transfer-DNA (T-DNA) region, is transferred to the plant and leads to the induction of tumours. The other region, the virulence-imparting (vir) region, is essential only for the formation of the tumours, not for their maintenance. The dimensions of the transfer-DNA region can be enlarged by incorporation of the mutated histidinol dehydrogenase-encoding DNA sequence without the transferability being impaired. By removing the tumour-inducing genes and incorporating a selectable marker, the modified Ti- or Ri-plasmid can be used as a vector for the transfer of the gene construction according to the invention into a suitable plant cell.

The vir region brings about the transfer of the T-DNA region of *Agrobacterium* to the genome of the plant cell irrespective of whether the T-DNA region and the vir region are present on the same vector or on different vectors within the same *Agrobacterium* cell. A vir region on a chromosome also induces the transfer of the T-DNA from a vector into a plant cell.

Thus, for transferring the histidinol dehydrogenase encoding DNA into plant cells a system can be used, in which the vir region and the T-DNA region are located on different vectors. Such a system is known as a "binary vector system", and the vector containing the T-DNA is accordingly designated a "binary vector".

Any T-DNA-containing vector that can be transferred into plant cells and permits selection of the transformed cells is suitable for use-within the scope of this invention such as, for example, a shuttle vector that comprises the mutated histidinol dehydrogenase-encoding DNA sequence according to the invention cloned in between the left border sequence (LB) and the right border sequence (RB) and that is capable of stable replication both in *E. coli* and in *A. tumefaciens*.

Using newly developed transformation techniques, it has also become possible in principle to transform *in vitro* plant species that are not natural host plants for *Agrobacterium*. For example, monocotyledonous plants, especially the cereal species and various grasses, are not natural hosts for *Agrobacterium*.

It has become increasingly evident that monocotyledons can also be transformed using *Agrobacterium*, so that, using new experimental formulations that are now becoming available, cereals and grass species are also amenable to transformation [Grimsley NH *et al* (1987)].

One of the preferred methods for introducing DNA into a plant cell by means of *Agrobacterium* is the so-called leaf disk transformation using *Agrobacterium* [Horsch *et al* (1985)]. Sterile leaf disks from a suitable target plant are incubated with *Agrobacterium* cells comprising one of the mutated histidinol dehydrogenase-encoding DNA sequence according to the invention, and are then transferred into or onto a suitable nutrient medium. Especially suitable, and therefore preferred within the scope of this invention, are LS media that have been solidified by the addition of agar and enriched with one or more of the plant growth regulators customarily used, especially those selected from the group of the auxins consisting of a-naphthylacetic acid, picloram, 2,4,5-trichlorophenoxyacetic acid, 2,4-dichlorophenoxyacetic acid, indole-3-butyric acid, indole-3-lactic acid, indole-3-succinic acid, indole-3-acetic acid and p-chlorophenoxyacetic acid, and from the group of the cytokinins consisting of kinetin, 6-benzyladenine, 2-isopentenyladenine and zeatin. The preferred concentration of auxins and cytokinins is in the range of from 0.1 mg/l to 10 mg/l.

After incubation for several days, but preferably after incubation for 2 to 3 days at a temperature of from 20°C to 40°C, preferably from 23°C to 35°C and more especially at 25°C and in diffuse light, the leaf disks are transferred to a suitable medium for the purpose of shoot induction. Especially preferred for the selection of the transformants is an LS medium that does not contain auxin but contains cytokinin instead, and to which a selective substance has been added dependent on the marker gene used. The cultures are kept in the light and are transferred to fresh medium at suitable intervals, but preferably at intervals of one week. Developing green shoots are cut out and cultured further in a medium that induces the shoots to form roots. Especially preferred within the scope of this invention is an LS medium that does not contain auxin or cytokinin but to which a selective substance has been added for the selection of the transformants.

In addition to *Agrobacterium*-mediated transformation, within the scope of this invention it is possible to use direct transformation methods for the insertion of the gene constructions according to the invention into plant material.

Possible methods for the direct transfer of genetic material into a plant cell comprise, for example, the treatment of protoplasts using procedures that modify the plasma membrane, for example polyethylene glycol treatment, heat shock treatment or electroporation, or a combination of those procedures [Shillito *et al* (1985)].

In the electroporation technique, plant protoplasts together with plasmids that comprise the mutated histidinol dehydrogenase-encoding DNA sequence are subjected to electrical pulses of high field strength. This results in a reversible increase in the permeability of biomembranes and thus allows the insertion of the plasmids. Electroporated plant protoplasts renew their cell wall, divide and form callus tissue. Selection of the transformed plant cells can take place with the aid of the above-described phenotypic markers.

A further method for the direct introduction of genetic material into plant cells, which is based on purely chemical procedures and which enables the transformation to be carried out very efficiently and rapidly, is described in Negrutiu I *et al* (1987).

Also suitable for the transformation of plant material is direct gene transfer using co-transformation (Schocher RJ *et al* 1986).

Co-transformation is a method that is based on the simultaneous taking up and integration of various DNA molecules (non-selectable and selectable genes) into the plant genome and that therefore allows the detection of cells that have been transformed with non-selectable genes.

Further means for inserting genetic material contained in a vector directly into a plant cell comprise using purely physical procedures, for example by microinjection using finely drawn micropipettes [Neuhaus *et al* (1987)] or by bombarding the cells with microprojectiles that are coated with the transforming DNA ["Microprojectile Bombardment"; Wang Y-C *et al* (1988)] or are accelerated through a DNA containing soluting in the direction of the cells to be transformed by a pressure impact thereby being finly atomized into a fog with the solution as a result of the pressure impact [EP-A 434 616].

Microprojectile bombardment has been advanced as an effective transformation technique for cells, including cells of plants. In Sanford *et al* (1987) it was reported that microprojectile bombardment was effective to deliver nucleic acid into the cytoplasm of plant cells of *Allium cepa* (onion). Christou *et al* (1988) reported the stable transformation of soybean callus with a kanamycin resistance gene *via* microprojectile bombardment. Christou *et al* reported penetration at approximately 0.1 % to 5 % of cells. Christou further reported observable levels of NPTII enzyme activity and resistance in the transformed calli of up to 400 mg/L of kanamycin. McCabe *et al* (1988) report the stable transformation of *Glycine max* (soybean) using microprojectile bombardment. McCabe *et al* further report the recovery of a transformed $R_1$ plant from an $R_0$ chimeric plant.

The transformation of maize plants, including elite maize plants, by microprojectile bombardment can be carried out according to the following general protocol:

Maize plants are transformed using a suitable vector molecule comprising the genes for histidinol dehydrogenase and preferably a scorable and/or a selectable marker. An example of a suitable vector is a plasmid comprising a double CaMV 35S promoter/-terminator cassette containing a selectable marker such as, for example, an antibiotic resistance, e.g. ampicillin or chloramphenicol resistance.

Preferred is plasmid pCGN1761 [see EXAMPLE 14 for its construction] which contains a double CaMV 35 S promoter and the tml-3' region with an EcoRI site between contained in a pUC-derived plasmid backbone. The promoter-EcoRI-3' processing site cassette is bordered by multiple restriction sites for easy removal. The plasmid is derived by a series of steps (see below) from an initial double-35S plasmid, pCGN2113, which itself is derived from pCGN164, and pCGN638. The plasmid pCGN2113 is deposited with ATCC on March 22, 1989, accession number 40587.

The target for microprojectile bombardment may be tassel primordia, shoot meristems, zygotic embryos or, preferably, a friable, Type II embryogenic maize culture. Each target preferably contains approximately 250 milligrams of this culture plated onto filters such as, for example, Durapore filters, which are placed on top of suitable medium. An example of a suitable medium is a N6 media of Chu *et al* (1975) supplemented with 3 percent sucrose and 2 milligrams per liter of 2,4-dichlorophenoxyacetic acid, which is designated '2N63S'.

Preferentially a 2X gene dose of GUS gene 'equivalents' of the plasmids pCHN1761, wherein the histidinol dehydrogenase gene is placed into the vector, or the histidinol dehydrogenase clones contained therein and plasmids containing selectable markers such as phosphinothricin resistance, hygromycin resistance, paromomycin resistance or scorable markers such as glucouronidase (GUS) or luciferase are co-precipitated onto tungsten particles according to the $CaCl_2$-Spermidine precipitation method of Klein *et al* (1988). Five microliters of the DNA-tungsten preparation is pipetted onto the macroprojectile and propelled toward the target using a number 1 gray cartridge essentially as described in the operating instructions for the bombardment device.

After 5 days, the cells are transferred to fresh filters and placed onto media containing selection agents at a concentration of 1 to 500 milligrams of per liter, depending on the agent used. The filters with the cells are transferred approximately weekly to fresh medium containing the selection agent. The concentration of the agent may be increased during the course of selection. After 6 weeks of selection, colonies appeared randomly on the filters. Colonies are isolated over the course of three weeks, each one being transferred to fresh medium containing the selection agent but without a filter for a secondary selection period.

The Type II embryogenic callus cultures employed as the target for transformation *via* microprojectile bombardment, may become established using one of several explant sources. Suitable explant sources are, for example, immature embryos, immature tassels, leaf bases, apical meristems of either roots or shoots, immature ear shoots, anthers or microspores, and ovules. Preferred sources are apical meristems, leaf bases, immature embryos and immature tassels. Most preferred sources are immature embryos and immature tassels.

Media which are capable of initiating Type II embryogenesis are those comprising an inorganic salts formulation similar to that present in the medium of Schenk and Hildebrandt (1972), but with a greater nitrogen content and lower nitrate:ammonium ion ratio than that employed by Schenk and Hildebrandt.

In particular, the media comprise microelement concentrations similar to those employed in the basal media of Schenk and Hildebrandt. The media further comprise a total nitrogen content of from about 40 to about 80 mM; most preferably about 60 mM; a nitrate:ammonium ion ratio of less than about 4.0, most preferably about 1.9; and a total sulfate concentration of about 1.6 to about 8.6 mM, more preferably from about 1.6 to about 4.6 mM. The media further include a carbon nutrient source, preferably sucrose. The sucrose is preferably present in amounts from about 1 to about 6 % weight per volume, most preferably 2 %. The nutrient source, other than sucrose, is preferably present in the molar equivalent of the above sucrose concentrations. The media preferably further comprise an auxin. The auxin is preferably 3,6 dichloroortho anisic acid, known as dicamba. The concentration of the auxin is preferably from about 0.1 to about 10 mg/l, more preferably from about 0.5 to about 1 mg/l. The media also preferably includes a solidifying agent. Preferred solidifying agents include Noble Agar, Phytagar, and Gelrite, especially 0.8 % Phytagar™ and 0.24 % Gelrite™. Utilizing this media formulation, it is possible to initiate Type II embryogenic callus in maize lines, including elite maize lines, with a frequency of greater than about 2 %, preferably greater than about 10 %, and most preferably with a frequency greater than about 20 %. Utilizing the above media, it is further possible to obtain Type II embryogenic response within a period of about 14 days, and to regenerate whole plants from maize lines, including elite maize lines, with a frequency beyond that obtained with known media formulations.

Type II embryogenic callus is identified by its morphology. It has been defined as "friable and fast growing [callus] with well defined somatic embryos with suspensor-like structures" [Tomes *et al*, 1985] or as "friable, embryogenic" [Armstrong *et al*, US-P 4,761.373]. The nature of Type II embryogenic callus is such that it possesses a greater quantity of cells individually competent for embryogenesis and regeneration, are easier to manipulate when exposed to selection agents in the culture media, are more amenable as targets for transformation, and are used as the starting material for suspension and protoplast cultures. The visual appearance of Type II embryogenic callus is shown in Tomes *et al* (1985)("Tomes 2").

Thus, the media useful for transformation are those that will induce the formation of Type II embryogenic callus, as described further herein. Type II embryogenic callus cultures are desirable because they are the starting point for embryogenic suspension cultures and regenerable protoplast cultures. Typically, the newly initiated Type II response is established by subculturing on an appropriate media, during which time its friability and growth are improved. Regeneration potential is maintained during this subculture process.

Screening of maize cells, tissue and plants for the presence of specific DNA sequences is performed by Southern analysis [Southern, (1975)]. Details of this procedure are given in Maniatis *et al* (1983). This screening can also be performed by the use of Polymerase Chain Reaction procedures (PCR). PCR procedures are described in detail in Mullis *et al* (1987) and Erlich, (1989). The specific primers employed in this invention are further described in EXAMPLE 10.

Transformation of the plant cells includes separating transformed cells from those that have not been transformed. One convenient method for such separation or selection is to incorporate into the material to be inserted into the transformed cell a gene for a selection marker. As a result only those cells that have been successfully transformed will contain the marker gene. The translation product of the marker gene will then confer a phenotypic trait that will make selection possible. Usually the phenotypic trait is the ability to survive in the presence of some chemical agent, such as an antibiotic, e.g., kanamycin, G418, paromomycin, etc., which is placed in a selection media.

Some examples of genes that confer antibiotic resistance include, for example, those coding for neomycin phosphotransferase [kanamycin resistance, Velten *et al* (1984)]; hygromycin phosphotransferase [hygromycin resistance, van den Elzen *et al.*, Plant Molecular Biology 5: 299-392 (1985)], the kanamycin resistance (NPT II) gene derived from Tn5 [Bevan *et al* (1983); McBride *et al* (1990)), the PAT gene described in Thompson *et al* (1987), and chloramephenicol acetyltransferase.

An example of a gene useful primarily as a screenable marker in tissue culture for identification-of plant cells containing genetically engineered vectors is a gene that encodes an enzyme producing a chromogenic product. One example is the gene encoding for production of beta-glucuronidase (GUS). This enzyme is widely used and its preparation and use is described in Jefferson (1987).

Once the transformed plant cells have been cultured on the selection media, surviving cells are selected for further study and manipulation. Selection methods and materials are well known to those of skill in the art, allowing one to choose surviving cells with a high degree of predictability that the chosen cells will have been successfully transformed with exogenous DNA.

After transformation of the plant cell or plant using, for example, the *Agrobacterium* Ti-plasmid, those plant cells or plants transformed by the Ti plasmid so that the enzyme is expressed, can be selected by an appropriate phenotypic marker. These phenotypical markers include, but are not limited to, antibiotic resistance. Other phenotypic markers are known in the art and may be used.

All plants from which protoplasts can be isolated and cultured to give whole regenerated plants can be transformed so that whole plants are recovered which contain the DNA coding sequence. Some suitable plants include, for example, species from the genera *Fragaria, Lotus, Medicago, Onobrychis, Trifolium, Trigonella, Vigna, Citrus, Linum, Geranium, Manicot, Daucus, Arabidopsis, Brassica, Raphanus, Sinapis, Atropa, Capsicum, Datura, Hyoscyamus, Lycopersion, Nicotiana, Solanum, Petunia, Dactylis, Majorana, Cichorium, Helianthus, Lactuca, Bromus, Asparagus, Antirrhinum, Hemerocallis, Nemesia, Pelargonium, Panicum, Pennisetum, Ranunculus, Senecio, Salpiglossis, Cucumis, Browallia, Glycine, Lolium, Zea, Triticum, Sorghum and Datura.*

There is an increasing body of evidence that practically all plants can be regenerated from cultured cells or tissues, including but not limited to, all major cereal crop species, sugarcane, sugar beet, cotton, fruit and other trees, legumes and vegetables. Limited knowledge presently exists on whether all of these plants can be transformed by *Agrobacterium*. Species which are a natural plant host for *Agrobacterium* may be transformable *in vitro.* Monocotyledonous plants, and in particular, cereals and grasses, are not natural hosts to *Agrobacterium*. Attempts to transform them using *Agrobacterium* have been successful recently [Hooykas-Van Slogteren *et al* (1984)]. There is growing evidence now that certain monocots can be transformed by *Agrobacterium*. Using current experimental approaches that have now become available, cereal and grass species may be transformable [Grimsley *et al*, EP-A 0 267 159] by *Agrobacterium*.

Additional plant genera that may be transformed by *Agrobacterium* include *Ipomoea, Passiflora, Cyclamen, Malus, Prunus, Rosa, Rubus, Populus, Santalum, Allium, Lilium, Narcissus, Ananas, Arachis, Phaseolus and Pisum*.

Plant regeneration from cultural protoplasts is described in Evans *et al* (1983); M.R. Davey (1983); P.J. Dale (1983); and H. Binding (1985).

Regeneration varies from species to species of plants, but generally a suspension of transformed protoplasts containing multiple copies of the toxin gene is first provided. Embryo formation can then be induced from the protoplast suspensions, to the stage of ripening and germination as natural embryos. The culture media will generally contain various amino acids and hormones, such as auxin and cytokinins. It is also advantageous to add glutamic acid and proline to the medium, especially for such species as corn and alfalfa. Shoots and roots normally develop simultaneously. Efficient regeneration will depend on the medium, on the genotype, and on the history of the culture. If these three variables are controlled, then regeneration is fully reproducible and repeatable.

The mature plants, grown from the transformed plant cells, are selfed to produce an inbred plant. The inbred plant produces seed containing the hybrid gene. These seeds can be grown to produce plants that have the hybrid gene. Such transformed plants and seeds are known as transgenic plants and seeds. Preferred transgenic plants are transgenic crops, which are plants suitable for human cultivation, more preferably plants all or part of which are fit for consumption by animals raised as a source of human food, e.g., livestock, poultry, fish, etc.

Such transformed seeds can then be used to improve crop yields by growing transformed plants from the transformed seeds in the presence of a histidinol dehydrogenase inhibitor that would be herbicidal to nontransformed plants growing among the transformed crops (i.e., weeds). Genetically engineering crops in this fashion to become resistant to a herbicide with no vertebrate toxicity would encourage the use of safe herbicides on this type as an alternative to more broadly toxic herbicides of questionable environmental safety and vertebrate toxicity.

The active ingredients of the present invention are normally applied in the form of compositions together with one or more agriculturally acceptable carriers, and can be applied to the crop area or plant to be treated, simultaneously or in succession, with further compounds. These compounds can be both fertilizers or micronutrient donors or other preparations that influence plant growth. They can also be selective herbicides, insecticides, fungicides, bactericides, nematicides, mollusicides or mixtures of several of these preparations, if desired together with further carriers, surfactants or application-promoting adjuvants customarily employed in the art of formulation. Suitable carriers and adjuvants can be solid or liquid and correspond to the substances ordinarily employed in formulation technology, e.g. natural or regenerated mineral substances, solvents, dispersants, wetting agents, tackifiers, binders or fertilizers.

A preferred method of applying active ingredients of the present invention or an agrochemical composition which contains at least one of the active ingredients is leaf application. The number of applications and the rate of application depend on the intensity of infestation by the corresponding weed. However, the active ingredients can also penetrate the plant through the roots via the soil (systemic action) by impregnating the locus of the plant with a liquid composition, or by applying the compounds in solid form to the soil, e.g. in granular form (soil application). The active ingredients may also be applied to seeds (coating) by impregnating the seeds either with a liquid formulation containing active ingredients, or coating them with a solid formulation. In special cases, further types of application are also possible, for example, selective treatment of the plant stems or buds.

The active ingredients are used in unmodified form or, preferably, together with the adjuvants conventionally employed in the art of formulation, and are therefore formulated in known manner to emulsifiable concentrates, coatable pastes, directly sprayable or dilutable solutions, dilute emulsions, wettable powders, soluble powders, dusts, granulates, and also encapsulations, for example, in polymer substances. Like the nature of the compositions, the methods of application, such as spraying, atomizing, dusting, scattering or pouring, are chosen in accordance with the intended objectives and the prevailing circumstances. Advantageous rates of application are normally from 50 g to 5 kg of active ingredient (a.i.) per hectare ("ha", approximately 2.471 acres), preferably from 100 g to 2 kg a.i./ha, most preferably from 200 g to 500 g a.i./ha.

The formulations, compositions or preparations containing the active ingredients and, where appropriate, a solid or liquid adjuvant, are prepared in known manner; for example by homogeneously mixing and/or grinding the active ingredients with extenders, for example solvents, solid carriers and, where appropriate, surface-active compounds (surfactants).

Suitable solvents include aromatic hydrocarbons, preferably the fractions having 8 to 12 carbon atoms, for example, xylene mixtures or substituted naphthalenes, phthalates such as dibutyl phthalate or dioctyl phthalate, aliphatic hydrocarbons such as cyclohexane or paraffins, alcohols and glycols and their ethers and esters, such as ethanol, ethylene glycol monomethyl or monoethyl ether, ketones such as cyclohexanone, strongly polar solvents such as N-methyl-2-pyrrolidone, dimethyl sulfoxide or dimethyl formamide, as well as epoxidized vegetable oils such as epoxidized coconut oil or soybean oil; or water.

The solid carriers used e.g. for dusts and dispersible powders, are normally natural mineral fillers such as calcite, talcum, kaolin, montmorillonite or attapulgite. In order to improve the physical properties it is also possible to add highly dispersed silicic acid or highly dispersed absorbent polymers. Suitable granulated adsorptive carriers are porous types, for example pumice, broken brick, sepiolite or bentonite; and suitable nonsorbent carriers are materials such as calcite or sand. In addition, a great number of pregranulated materials of inorganic or organic nature can be used, e.g. especially dolomite or pulverized plant residues.

Depending on the nature of the active ingredient to be used in the formulation, suitable surface-active compounds are nonionic, cationic and/or anionic surfactants having good emulsifying, dispersing and wetting properties. The term "surfactants" will also be understood as comprising mixtures of surfactants.

Suitable anionic surfactants can be both water-soluble soaps and water-soluble synthetic surface-active compounds.

Suitable soaps are the alkali metal salts, alkaline earth metal salts or unsubstituted or substituted ammonium salts of higher fatty acids (chains of 10 to 22 carbon atoms), for example the sodium or potassium salts of oleic or stearic acid, or of natural fatty acid mixtures which can be obtained for example from coconut oil or tallow oil. The fatty acid methyltaurin salts may also be used.

More frequently, however, so-called synthetic surfactants are used, especially fatty sulfonates, fatty sulfates, sulfonated benzimidazole derivatives or alkylarylsulfonates.

The fatty sulfonates or sulfates are usually in the form of alkali metal salts, alkaline earth metal salts or unsubstituted or substituted ammoniums salts and have a 8 to 22 carbon alkyl radical which also includes the alkyl moiety of alkyl radicals, for example, the sodium or calcium salt of lignonsulfonic acid, of dodecylsulfate or of a mixture of fatty alcohol sulfates obtained from natural fatty acids. These compounds also comprise the salts of sulfuric acid esters and sulfonic acids of fatty alcohol/ethylene oxide adducts. The sulfonated benzimidazole derivatives preferably contain 2 sulfonic acid groups and one fatty acid radical containing 8 to 22 carbon atoms. Examples of alkylarylsulfonates are the sodium, calcium or triethanolamine salts of dodecylbenzenesulfonic acid, dibutylnapthalenesulfonic acid, or of a naphthalenesulfonic acid/formaldehyde condensation product. Also suitable are corresponding phosphates, e.g. salts of the phosphoric acid ester of an adduct of p-nonylphenol with 4 to 14 moles of ethylene oxide.

Non-ionic surfactants are preferably polyglycol ether derivatives of aliphatic or cycloaliphatic alcohols, or saturated or unsaturated fatty acids and alkylphenols, said derivatives containing 3 to 30 glycol ether groups and 8 to 20 carbon atoms in the (aliphatic) hydrocarbon moiety and 6 to 18 carbon atoms in the alkyl moiety of the alkylphenols.

Further suitable non-ionic surfactants are the water-soluble adducts of polyethylene oxide with polypropylene glycol, ethylenediamine propylene glycol and alkylpolypropylene glycol containing 1 to 10 carbon atoms in the alkyl chain, which adducts contain 20 to 250 ethylene glycol ether groups and 10 to 100 propylene glycol ether groups. These compounds usually contain 1 to 5 ethylene glycol units per propylene glycol unit.

Representative examples of non-ionic surfactants re nonylphenolpolyethoxyethanols, castor oil polyglycol ethers, polypropylene/polyethylene oxide adducts, tributylphenoxypolyethoxyethanol, polyethylene glycol and octylphenoxyethoxyethanol. Fatty acid esters of polyoxyethylene sorbitan and polyoxyethylene sorbitan trioleate are also suitable non-ionic surfactants.

Cationic surfactants are preferably quaternary ammonium salts which have, as N-substituent, at least one $C_8$-$C_{22}$alkyl radical and, as further substituents, lower unsubstituted or halogenated alkyl, benzyl or lower hydroxyalkyl radicals. The salts are preferably in the form of halides, methylsulfates or ethylsulfates, e.g. stearyltrimethylammonium chloride or benzyldi(2-chloroethyl)ethylammonium bromide.

The surfactants customarily employed in the art of formulation are described, for example, in "McCutcheon's Detergents and Emulsifiers Annual," MC Publishing Corp. Ringwood, New Jersey, 1979, and Sisely and Wood, "Encyclopedia of Surface Active Agents," Chemical Publishing Co., Inc. New York, 1980.

The agrochemical compositions usually contain from about 0.1 to about 99 %, preferably about 0.1 to about 95 %, and most preferably from about 3 to about 90 % of the active ingredient, from about 1 to about 99.9 %, preferably from about 1 to about 99 %, and most preferably from about 5 to about 95 % of a solid or liquid adjuvant, and from about 0 to about 25 %, preferably about 0.1 to about 25 %, and most preferably from about 0.1 to about 20 % of a surfactant.

Whereas commercial products are preferably formulated as concentrates, the end user will normally employ dilute formulations.

In the following a short description of the figures is provided, reference to which is made within the description and the EXAMPLES.

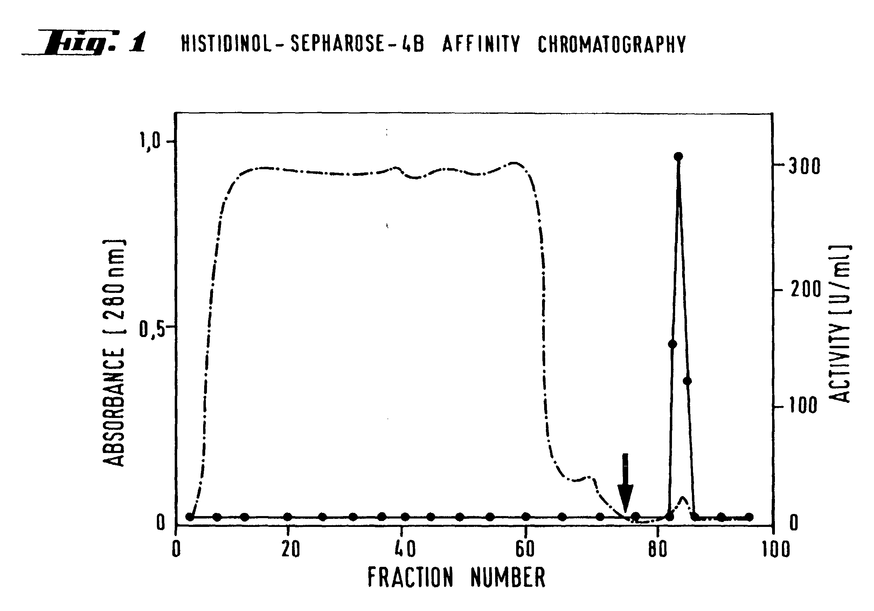

Figure 1: Histidinol-Sepharose-4B affinity chromatography. (------) Protein concentration represented by the absorption at 280nm; (o-o-o-) histidinol dehydrogenase activity expressed in U/ml. The affinity column was washed with 10mM Tris/HCl buffer, pH7.3 containing 140mM NaCl until all non-adsorbed protein was eluted (at fraction No. 75). The arrow at fraction No. 76 indicates the start of the elution with 50mM Tris/HCl buffer, pH 7.3 containing 700mM imidazole.

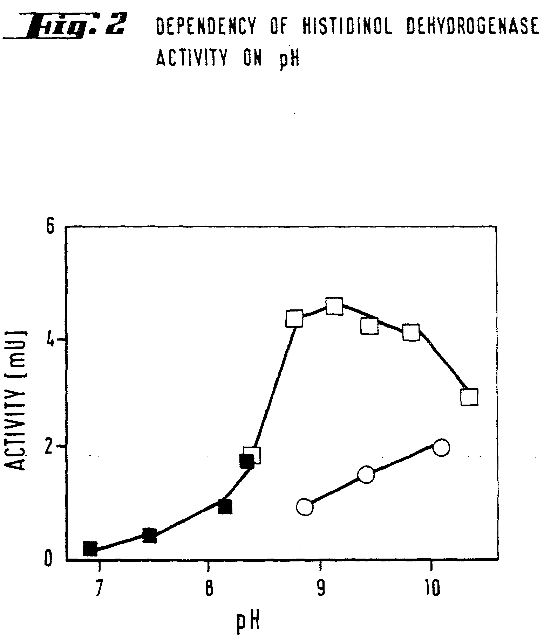

Figure 2: Dependency of histidinol dehydrogenase activity on pH. (■) reaction in 50mM HEPES buffer; ( O ) reaction in 50mM Gly/NaOH buffer, (□) reaction in 50mM CHES buffer. In all buffers, 0.5mM $MnCl_2$ was included. The reaction was performed under standard conditions as described in Materials and Methods. In 50mM phosphate buffer, pH7.5, no activity was detected.

SEQ ID NO: 1: The amino acid and nucleotide sequence for histidinol dehydrogenase from cabbage. Amino acids are represented by the three letter designations, nucleotides are represented by the standard letter designations where A is adenine, T is thymidine, C is cytosine, G is guanine, H is selected from thymidine or cytosine, and Y is selected from adenine, thymidine and cytosine.

SEQ ID NO: 2/3: The nucleotide sequence for histidinol dehydrogenase from wheat. Nucleotides are represented by the standard letter designations where A is adenine, T is thymidine, C is cytosine, G is guanine, H is selected from thymidine or cytosine, and Y is selected from adenine, thymidine and cytosine.

## EXAMPLES

The following examples are intended to exemplify specific embodiments of the present invention without limiting the scope in any way.

EXAMPLE 1: **Materials and Strains**

Sepharose-4B is purchased from Pharmacia LKB. Histidinol dihydrochloride is from Aldrich (Milwaukee, Wisconsin), and $NAD^+$, sodium metaperiodate, epichlorohydrin and nitroblue tetrazolium are from Sigma (St. Louis, Missouri). Sodium cyanoborohydride is purchased from Nakarai, and phenazine methosulfate from Wako Pure Chemical Industries (Osaka, Japan). Grace's insect cell culture medium, fetal bovine serum, Yeastolate and lactalbumin hydrolysate were purchased from Difco Laboratories. Gentamycin sulfate was from Sigma. All other chemicals used are of analytical grade.

Cabbage (*Brassica oleracea*), cucumber (*Cucumis sativus*), asparagus (*Asparagus officinalis*), eggplant (*Solanum melongena*), lettuce (*Lactuca sativa*), and pimento (*Capsicum annum*) are cultivated in a growth chamber with 16h/8h light/dark cycle at 25°C during illumination period and 15°C during dark period. The relative humidity is constant at 80%. Cell cultures of Rosa "Paul's Scarlet" are cultured in suspension as previously described by Strauss *et al* (1985). Cell cultures of tobacco (*Nicotiana tabacum* L. cv Samsun NN) and suspension cultures of wheat (*Triticum aestivum* var Chinese Spring) are grown as described by Kumpaisal *et al* (1987); and Yamada and Sato, (1978). Mature spring cabbage (*Brassica oleracea* L. *var* capitata L.) is purchased from a local grocer. Wheat germ is obtained from Sigma (St. Louis, Missouri).

However, it goes without saying that besides the cell and suspension cultures specifically named in the above paragraph, any other suitable cultivated material can be used equally well as starting material for the preparation of enzyme extracts.

Spodoptera frugiperda (Sf9) cells can be purchased from the American Type Culture Collection (ATCC) in Rockville, Maryland, USA under the accession number CRL1711. Alternative cell lines that can also be used in the process according to the invention comprise those from *Spodoptera frugiperda* such as Sf21, from *Mamestra brassicae*, and the like. A kit comprising a combination of a wild-type *Autographs californica* nuclear polyhedrosis virus (AcNPV, baculovirus) and the baculovirus transfer vectors pAC700, pAc701, pAc702, pVL1392 and pVL1393 are commercially available from Invitrogen.

Sf9 cells were maintained as a monolayer culture in Grace's medium supplemented with 10 % (v/v) bovine fetal serum, 0.33 % (w/v) Yeastolate, 0.33 % (w/v) lactalbumin hydrolysate and 50 µg/ml of gentamycin sulfate at 27°C.

EXAMPLE 2: **Preparation of Enzyme Extract**

Two week old plants, whole cabbage heads or well grown cell tissues are used as the enzyme sources. The plant material is homogenized with a Polytron blender in cold 100 mM sodium phosphate buffer, pH 7.2 (buffer A), and the homogenate passed through a filtration cloth. After centrifugation at 30,000 x g for 20 min, the supernatant is fractionated with 45-60% saturation of ammonium sulfate on ice. The precipitate is collected by centrifugation, dissolved in buffer A and the solution desalted on Sephadex G-25. This protein enriched extract is used for the determination of histidinol dehydrogenase activity in various plants.

EXAMPLE 3: **Purification of Histidinol Dehydrogenase**

Spring cabbage heads are homogenized using a kitchen mixer and suspension processed as described above, except that desalting is performed by extensively dialyzing against 50 mM Tris/HCl buffer, pH7.4 (buffer B). All subsequent procedures are carried out at 4°C.

Dialyzed protein extract is applied to a DEAE-Toyopearl [TOSOH Ltd (Tokyo)] column equilibrated with buffer B. After washing the column with buffer B, protein is eluted with a linear gradient of buffer B containing sodium chloride (0-500 mM). The protein fraction with activity is eluted from the DEAE-Toyopearl column before the main protein peak, at a concentration of 150 mM sodium chloride in the buffer.

Fractions containing enzyme activity are pooled and directly applied to a histidinol-Sepharose-4B column (2.5x8cm) (EXAMPLE 7) at a flow rate of 36 ml/h. Unabsorbed protein is eluted by extensively washing with 10mM Tris/HCl buffer, pH 7.3 containing 140 mM NaCl (buffer C). Bound histidinol dehydrogenase is eluted with 50 mM Tris/HCl, pH 7.3 containing imidazole.

Several unsuccessful attempts are made to purify histidinol dehydrogenase from mature spring cabbage heads by conventional purification methods. The development of an affinity gel, highly specific for this enzyme finally made it possible to purify the protein in three steps with a high yield to apparent homogeneity (Table I). All activity is bound to the gel, probably very tightly, since only 40% of the activity could be eluted specifically with a one step addition of high concentration of imidazole (700 mM) in the elution buffer (Fig. 1). By this step alone, the specific enzyme activity is increased by a factor of 350 to 10.16 U/mg of protein, showing this chromatography to be very specific for histidinol dehydrogenase. The over-all 2116-fold purification does not include the first step of fractionated ammonium sulfate precipitation, since no activity could be detected in crude extract. A heavily loaded SDS polyacrylamide gel showed one major band.

After desalting all fractions on Sephadex G-25, the enzyme activity in each fraction is determined. Active fractions are pooled and concentrated on an Amicon ultrafiltration membrane (YM-10). This enzyme preparation is stored at -80°C and used for all experiments, except for the activity screening in plants.

Storage of the enzyme for more than 4 months at -80°C did not change significantly the activity. At 4°C, the enzyme lost 10% of its activity during one week. By the addition of glycerol to a final concentration of 20%, full activity could be maintained at 4°C for more than one week.

TABLE I:

Purification of histidinol dehydrogenase. In this typical purification, 10 kg of cabbage heads are processed. All activities are measured after desalting with either Sephadex G-25 or dialysis. The activity in the crude extract could not be detected (n.d.).

| Purification step | Total protein [mg] | Total activity [U] | Recovery activity [%] | Specific activity [U/mg] | Purity [-fold] |
| --- | --- | --- | --- | --- | --- |
| Crude extract | n.d. | n.d. | n.d. | n.d. | n.d. |
| Ammonium sulfat fractionation | 4750 | 23 | 100 | 0.0048 | 1 |
| DEAE-Toyopearl ionexchange chromatography | 560 | 20.1 | 87 | 0.029 | 6 |
| Histidinol-Sepharose-4B affinity chromatography | 0.75 | 7.6 | 33 | 10.16 | 2116 |

EXAMPLE 4: **Gel Filtration**

A Sepharose 12 column (Pharmacia LKB, Piscataway, New Jersey) is equilibrated with purification buffer C [see EXAMPLE 3] at room temperature. Samples of purified enzyme are applied to the column and eluted with buffer C at a flow rate of 0.5 ml/min. The molecular mass is calculated using a molecular weight marker kit (Sigma, St. Louis, Missouri). The native molecular weight of the enzyme is determined by Superose 12 gel filtration [an analytical gel filtration medium available from LKO Pharmacia, Piscataway, NJ, USA]to be 103,000.

EXAMPLE 5: **Polyacrylamide Gel Electrophoresis**

SDS-polyacrylamide gel electrophoresis is performed as described elsewhere [Laemmli (1970)], using 10-20 % acrylamide. Proteins in sample buffer [Tris-HCl (pH 6.8) 310 mM; SDS 1 %; glycerol 5 %; beta-mercaptoethanol 2.5 %; EDTA 1.24 mM; bromphenol blue 0.0005 %] are put in a heating block at 100°C for 10 min. For the calculation of the molecular mass of denatured and reduced protein, an electrophoresis calibration kit for molecular weight determination [Pharmacia, Piscataway, New Jersey] is used. Under denaturing and reducing conditions, the enzyme migrated as a single band of 52 kDaltons if exposed to SDS-polyacrylamide gel electrophoresis, suggesting a dimeric quaternary structure of the enzyme.

The molecular weight of 103,000 of native cabbage enzyme is considerably higher than that of the enzyme from *S. typhimurium* [82,000, Burger *et al* (1979)]; and 90,000, [Eccleston *et al* (1979)]). The enzymes from cabbage and *S. typhimurium* are each composed of two subunits with a similar molecular weight of 52,000 and 43,000 [Burger *et al* (1979)], respectively. However, the subunit molecular weight of the trifunctional enzyme from *S. cerevisiae* is calculated from the nucleotide sequence to be 87,935 [Donahue *et al* (1982)] and estimated on SDS gels as about 95,000 [Keesey *et al* (1979)]. According to these data, the enzymes from yeast, *S. typhimurium* and cabbage differ significantly in their molecular weights.

EXAMPLE 6: **Isoelectric Focusing**

PhastGel (Pharmacia LKB, Piscataway, New Jersey) with a pH-range of 4-6.5 is used for isoelectric focusing, performed on a Phast System (Pharmacia LKB, Piscataway, New Jersey) according to the instruction manual of the supplier. The isoelectric point of the protein is determined using an isoelectric point calibration kit (Pharmacia LKB, Piscataway, New Jersey).

The exposure of purified enzyme to analytical isoelectric focusing lead to its separation into six protein bands within the pH-range 5.1-5.4. Five bands showed histidinol dehydrogenase activity by employing activity staining. The sixth band had only very slight or no activity. In control experiments lacking either histidinol or $NAD^+$ in the reaction mixture, p-nitro blue tetrazolium is not reduced to formazan. The activity of each of the five bands correlated with their colour intensities obtained by protein staining.

To rule out artifacts of the purification procedure, several independent purifications are carried out, starting from cabbage heads in different growth stages. In all cases, six protein bands are obtained.

EXAMPLE 7: **Preparation of Histidinol-Sepharose-4B Gel**

Sepharose-4B is activated with epichlorohydrin as previously described [Matsumoto *et al* (1979)]. Epoxyactivated Sepharose-4B gel is suspended in 0.1 N NaOH containing 0.5g glucose per g of gel and incubated at 40°C on a shaker for 24h [Kanamori *et al* (1986)]. After washing extensively with water, the gel is suspended in pre-chilled sodium metaperiodate and the suspension shaken for 1h at 4°C. The gel is washed with water, suspended in 0.1 M HCl and incubated at room temperature for 1h. Again, the gel is washed extensively with water, followed by thorough washing with 10 mM sodium phosphate buffer, pH 7.0 at room temperature.

The formyl carrier gel is suspended in 10 mM sodium phosphate buffer, pH 7.0 containing 100 mM of histidinol dihydrochloride, which is previously neutralized with NaOH, and the suspension incubated at 4°C overnight. Five milligrams of sodium cyanoborohydride per g of gel is then added to the suspension and incubated with shaking at room temperature for 6h. The gel is extensively washed with distilled water and treated with 5mg sodium borohydride per g or gel for 3h at 4°C to convert the remaining formyl groups into hydroxymethyl groups.

EXAMPLE 8: **Enzyme Assay**

The enzyme activity is assayed spectrophotometrically by measuring the increase in absorbence at 340 nm due to the reduction of $NAD^+$ in a Hitachi U-3120 spectrophotometer. The reaction mixture contained 150 mM Gly/NaOH buffer, pH 9.2, 0.5 mM $MnCl_2$, 2 mM $NAD^+$, 5 mM histidinol, and 2-5 mU of enzyme sample in total volume of 0.5 ml. The reaction is started with the addition of histidinol, and incubated at 30°C. A reference sample containing water instead of histidinol is always run. One unit of enzyme catalyzing the formation of 25 M of NADH per min under the assay conditions.

In some experiments the activity is also followed by measuring the formation of His with a Hitachi L-850 amino acid analyzer. The enzyme reaction is terminated with formic acid (final concentration, 30%). The sample is then evaporated, and the precipitate dissolved in 0.2 N HCl. This preparation is used for His analysis.

Distribution of Histidinol Dehydrogenase in eleven plant species or preparations are examined for histidinol dehydrogenase activity. Enzyme activity could not be detected in any crude extract, but is found in enriched extracts from various monocotyledon and dicotyledon species (Table I). Cultured rose cells and spring cabbage showed the highest specific enzyme activities. Wheat germ had an extremely high extractable activity probably due to its low content of water, however, its specific activity is modest. Also shoots of cabbage and cucumber, and spring cabbage heads contained a high extractable activity. No activity could be detected in 2 week old pimento shoots and cultured tobacco cells.

Histidinol dehydrogenase in microorganisms catalyzes the two last reactions to form the endproduct His. To verify this catalytic role for the isolated protein from cabbage, its catalyzed reaction is analyzed stoichimetrically with regard to His formation and $NAD^+$ reduction. The amount of enzymatically produced NADH is determined from the absorbence at 340nm, using the absorbence coefficient of 6220. The other formed product is identified by amino acid analysis as His. Authentic His added to reacted sample is co-eluted with enzymatically formed His. Neither His nor NADH are formed in the absence of $NAD^+$, histidinol, or by using cooked enzyme. Two moles of NADH are formed for 0.99 mole of His after a reaction time of 2 min. (Table III). The formation of both products histidine and NADH is time dependent and correlated.

Optimum pH for the activity is at 9.2 in 50 mM Gly/NaOH buffer (Fig. 2). At pH values below 9, activity decreased in either Gly/NaOH buffer or in 50mM HEPES buffer. At pH 9.2-10, almost full activity is maintained, but above 10, a rapid loss of activity occurred.

The apparent $K_m$ values of the enzyme for L-histidinol and $NAD^+$ are determined under standard assay conditions such as 15.5 and 42 µM, respectively.

The influence of divalent metal ions on the enzyme reaction is shown in Table IV. The reaction is stimulated 26% by the addition of $Mn^{2+}$ compared to control conditions without the addition of any divalent metal ion to the reaction buffer (150 mM Gly/NaOH, pH9.2). The addition of $Ba^{2+}$, $Mg^{2+}$, $Ni^{2+}$, $Ca^{2+}$, $Zn^{2+}$ and $Cu^{2+}$ caused inhibition of the reaction.

Histidinol dehydrogenase from cabbage is the first His biosynthesis enzyme that has been purified to homogeneity from higher plants. By using a new affinity chromatography, the purification of this enzyme and its investigation became possible. Direct evidence that the purified protein is histidinol dehydrogenase is obtained from activity staining of the protein on the gel after isoelectric focusing. The enzymatically formed product starting from histidinol is identified as His. His and NADH are produced stoichiometrically, showing the same reduction of 2 mole of $NAD^+$ for 1 mole of formed His as previously described for this reaction in microorganisms [Adams (1955)]. The present results demonstrate that the last two reaction steps in the His biosynthetic pathway in cabbage are identical to those in microorganisms and are catalyzed by the same single enzyme. The $K_m$ of this enzyme for L-histidinol (15.5 µM at pH 9.2) is very similar to those (16 and 8.8 µM) of the pure enzyme from *S. typhimurium* [Burger *et al* (1981)] and the partially purified one from wheat germ [Wong and Mazelis (1981)]. The $K_m$ (42 µM) for $NAD^+$ is considerably lower than that of the enzymes from *S. typhimurium* (1 mM) (24) and from wheat germ (140 µM, using partially purified enzyme [Wong and Mazelis (1981)]). The pH-optimum of the reaction between pH 9.2-9.4 is identical to that of histidinol dehydrogenase from other organisms. No other divalent metal ion than $Mn^{2+}$ showed a stimulation of the enzyme activity. This is in line with the results obtained with the enzyme from *S. typhimurium*, whose catalyzed reaction is also stimulated in the presence of $Mn^{2+}$ [Grubmeyer *et al* (1989)].

TABLE II:

Distribution of histidinol dehydrogenase in different plant species and preparations. For the calculation of the extractable activity, the wet weight of the plant material is used. The weight of cells from submerse tissue cultures is determined after collection by filtration with a cloth.

*) mU/mg: units of activity per mg of protein;

**) mU/g: units of activity per g of plant material.

TABLE II

| Plant source | Specific Activity mU/mg*) | Extractable Activity mU/mg**) |
|---|---|---|
| Cabbage shoots (*Brassica oleracea* L. *var capitata* L.) | 1.6 | 3.3 |
| Asparagus shoots (*Asparagus officinalis*) | 0.9 | 1.6 |
| Lettuce shoots (*Lactuca saliva*) | 0.4 | 0.3 |
| Egg plants shoots (*Solanum melongena*) | 0.4 | 0.9 |
| Cucumber shoots (*Cucumis sativus*) | 0.3 | 3.3 |
| Pimento shoots (*Capsicum annum*) | 0 | 0 |
| Spring cabbage (*Brassica oleracea* L. *var capitata* L.) | 6.1 | 2.8 |
| Wheat germ | 2.1 | 91 |
| Wheat cell culture (*Triticum aestivum var* Chinese Spring) | 0.5 | 0.4 |
| Rose cell culture (Rosa "Paul's Scarlet") | 6.2 | 0.9 |
| Tobacco cell culture (*Nicotiana tabacum* L. cv Samsun (NN) | 0 | 0 |

TABLE III:

Stoichiometry of histidinol dehydrogenase reaction with regard to the formation of His and $NAD^+$. The enzyme reaction is stopped after 2, 5 and 30min reaction time and the sample analyzed by automated amino acid analysis. Histidinol does not react with ninhydrin and cannot be analyzed by amino acid analysis (at 50 nM).

TABLE III

| Reaction time min | His concentration µmole/ml (a) | NADA concentration µmole/ml (b) | b/a |
|---|---|---|---|
| 0 | 0 | 0 | - |
| 2 | 17.6 | 35.6 | 2.02 |

TABLE III   (continued)

| Reaction time min | His concentration μmole/ml (a) | NADA concentration μmole/ml (b) | b/a |
|---|---|---|---|
| 5 | 36.6 | 80.0 | 2.18 |
| 30 | 143.7 | 316.0 | 2.2 |

TABLE IV:

Effect of divalent metal ions on histidinol dehydrogenase activity. The concentration of metal ion is 0.5mM. The activity is determined after 3min reaction time, while the velocity is still constant.

TABLE IV

| Addition | Relative Activity % |
|---|---|
| None | 100 |
| $Mn^{2+}$ | 126 |
| $Ba^{2+}$ | 65 |
| $Mg^{2+}$ | 54 |
| $Ni^{2+}$ | 46 |
| $Ca^{2+}$ | 36 |
| $Zn^{2+}$ | 34 |
| $Cu^{2+}$ | 24 |

EXAMPLE 9: **Activity Staining**

Isoelectric point calibration proteins and purified enzyme (in quadruplicate) are applied on a PhastGel (pH-range 4-6.5) to give five lanes. After running the isoelectric focusing, the gel is cut into four pieces. The one containing marker proteins in addition to enzyme is stained with Coomassie Brilliant Blue R250. Another piece of gel containing only enzyme is subjected to activity staining as described elsewhere [Skyring *et al* (1970)], with a slight modification. The gel is immersed in 10 ml of reaction mixture containing 5 mM histidinol and 2 mM $NAD^+$. By the addition of 150 μl of nitroblue tetrazolium and 150 μl of phenazine methosulfate to give a final concentration of 0.5 mM and 0.13 mM, respectively, the staining process is started. Incubation is carried out at 30°C for 30 min. Two other pieces of gel containing only enzyme are identically incubated, but either without histidinol or without $NAD^+$.

EXAMPLE 10: **Protein Determination**

The concentration of the protein is determined by Bradford protein assay method using bovine serum albumin as a standard [Bradford (1976)].

EXAMPLE 11: **Amino Acid Sequencing**

Approximately 50 μg purified histidinol dehydrogenase is dried and resuspended in 0.1% trifluoroacetic acid and is subjected directly to automated Edman degradation with an Applied Biosystems 477A gas-liquid-phase protein sequencer [Strickler *et al* (1984)]. The phenylthiohydantion (PTH) amino acid derivatives are separated and identified with an on-line PTH analyzer (Applied Biosystems, Foster City, California) with PTH-$C_{18}$ column.
The first sequence run showed a recovery of 15% of the protein concentration determined by amino acid analysis, indicating that 85% of the N-terminus is blocked for unknown reason. The first N-terminal amino acid of the 15% of unblocked enzyme could not be determined unambiguously in both experiments. Ala and Lys are found most abundantly, but also Ser, Gly, Glu, Val and Leu are detected, all in similar concentrations.
The remainder of the N-terminal amino acid sequence is determined to be:
Met Lys Ile Tyr Arg Leu Ser Glu Leu Ser Phe ? Gln Val Glu Asn Leu Lys Ala Arg ?        ? Ile Asp
where question marks indicate residues that could not be determined from the amino acid sequencing data.
A further 160 μg of purified histidinol dehydrogenase protein is dried and resuspended in 415 μl of 20 mM Tris-HCl, pH 8.0. Lysyl endopeptidase [EC 3.4.21.50; Wako Chemicals, Osaka, Japan] is added to achieve a final concentration of approximately 33 μg/ml. This is equivalent to a weight ratio of approximately 1:500, peptidase:protein substrate. The digestion is incubated at room temperature for 24 hours, and the resulting peptides are separated by reverse phase HPLC on an Aquapore RP-300 (C-8) column (2.1 x 220 mm). The column is eluted with a 45 minute linear gradient of 0 - 70% acetonitrile in 0.1% TFA at a flow rate of 220 µl/minute. Peptides eluting at 17, 18, 32, 45, and 48 minutes are collected and analyzed by automated Edman degradation as described above. The following amino acid sequences are obtained:

```
Lys-C #17     Thr  Glu  Leu  Ser  Phe  Ala  Lys
Lys-C #18     Thr  Val  Val  Leu  Ala  Thr  Pro  Pro  Thr  Lys
Lys-C #32     Glu  Ala  Phe  Asp  Val  Ala  Tyr  Asp  Asn  Ile  Tyr  Ala
              Phe  His  Leu  Ala  Gln  Lys
Lys-C #45     Lys  Phe  Met  Thr  Val  Gln  Ser  Leu  Thr  Glu  Glu  Gly
              Leu  Arg  Asn  Leu  Gly  Pro  Tyr  Val  Ala  Thr  Met  Ala
              Glu  Ile  Glu  Gly  Leu  Asp  Ala
Lys-C #48     Ala  Leu  Ser  His  Ser  Phe  Thr  Val  Phe  Ala  Arg  Asp
              Met  Ile  Glu  Ala  Ile  Thr  Phe  Ser  Asn  Leu  Tyr  Ala
              Pro  Glu  Lys
```

A further 90 µg of purified histidinol dehydrogenase protein is dried and resuspended in 200 µl of 2% CNBr in 70% formic acid. The protein is digested at room temperature for 24 hours. The resulting peptides are purified exactly as described above, except that a 0 - 100% gradient of acetonitrle is used. Peptides eluting at 6 and 18 minutes are collected and subjected to amino acid sequencing by automated Edman degradation as described above. The following sequences are obtained:

```
CNBr # 6      Met  Ala  Glu  Ile  Glu  Gly  Leu  Asp  Ala  His  Lys  Arg
              Ala  Val  Thr  Leu  Arg  ?    Lys  Asp  Ile  Glu
CNBr #18      Leu  Ala  Ile  Pro  Ala  Gln  Ile  Ala  Gly  Arg  Lys  Thr
              Val  Val  Leu  Ala  Thr  Pro
```

EXAMPLE 12: **Obtaining the cDNA Sequence for Histidinol Dehydrogenase**

Three oligonucleotides that correspond to portions of the above amino acid sequences are synthesized by automated phosphoramidite synthesis on an Applied Biosystems (Foster City, California) Model 380B DNA Synthesizer.
Their sequences and the amino acid residues they correspond to are indicated below:

```
EW25  5'   TTAAGAATTCTAYGAYAAYATHTAYGC 3'
JR03  5'   CCYTCDATYTCNGCCAT 3'
JR02  5'   ATNGCYTCDATCATRTC 3'
```

where D indicates any the bases A, T, or G; H indicates the bases A, T, or C; R indicates the purines A or G; Y indicates the pyrimidines T or C. EW25 is a mixture of 48 oligonucleotides comprising all possible DNA sequences coding for the amino acid sequence Tyr Asp Asn Ile Tyr Ala, found within peptide Lys-C #32. JR03 is a mixture of 48 oligonucleotides comprising the complements of all possible DNA sequences coding for the amino acid sequence Met Ala Glu Ile Glu Gly, found within both peptides Lys-C #45 and CNBr #6. JR02 is a mixture of 48 oligonucleotides comprising the complementary strands of all possible DNA sequences coding for the amino acid sequence Asp Met Ile Glu Ala Ile, found within peptide Lys-C #48.
The sequences for the cabbage peptides are aligned with the translated sequence for the yeast HIS4C gene [Donahue *et al* (1982)], which encodes histidinol dehydrogenase. The cabbage peptides are found to have approximately 45% identity to the yeast sequence in their shared regions. Using such an alignment, and assuming that the cabbage sequence is approximately co-linear with the yeast sequence, Lys-C #32 is found to lie upstream (i.e. toward the N-terminus of the protein) relative to Lys-C #45 and CNBr #6, which lie near the C-terminus of the protein approximately 320 amino acids downstream. Lys-C #48 is found to lie between these peptides. Thus, EW25 should be relatively near the 5' end of the cDNA, JR03 should be near the 3' end of the cDNA, and JR02 should lie between these oligonuceotides.

Total RNA is prepared from frozen cabbage leaf tissue by phenol extraction followed by lithium chloride precipitation as described by Lagrimini et al. [Proc. Natl. Acad. Sci. USA 84, 7542-7546 (1987)]. Poly(A)+ mRNA is isolated from the total RNA using a poly(A) quick mRNA isolation kit (Stratagene, La Jolla, California) exactly as described in the manufacturer's instructions. The mRNA is enzymatically converted into cDNA and cloned in the lambda ZAP II vector using a ZAP-cDNA gigapack II gold synthesis kit (Stratagene, La Jolla, California) using instructions supplied with the kit by the manufacturer. The resulting cDNA library is propagated and amplified as described by the manufacturer of the kit.

Total DNA is prepared from the library using the Lambdasorb reagent (Promega Biotech, Madison, Wisconsin) by the method described by the manufacturer. A polymerase chain reaction [PCR; Saiki et al (1988)] is performed using the library DNA as template, and EW25 and JR03 as primers. The reaction is performed in a total volume of 50 µl, with 10 µg of template DNA, 200 pmole of each primer mixture, 100 µM each of dATP, dCTP, dGTP, and dTTP, 1x PCR buffer supplied by the manufacturer (Perkin Elmer Cetus, Norwalk, Connecticut) and 2.5 U AmpliTaq DNA polymerase (Perkin Elmer Cetus). The reaction is cycled through the following temperature profile 40 times, in a DNA Thermocycler (Perkin Elmer Cetus): 94°C for 45 seconds, 42°C for 45 seconds, and 72°C for 45 seconds. At each cycle, the 72°C step is increased by 2 seconds.

The PCR products are separated on a 2% low-gelling-temperature agarose gel (Nu-Sieve, FMC BioProducts, Rockland, Maine). Several fragments are detected by ethidium bromide stain, one of which is approximately 975 bp in length, the approximate size expected The gel is blotted to nylon membrane (GeneScreen Plus, NEN Research Products, Boston, MA) in 0.4 M NaOH as described by Reed and Mann (1985). The gel blot is hybridized to the JR02 oligonucleotide mixture which has been $^{32}$P-labeled using T4 polynucleotide kinase (New England Biolabs, Beverly, MA) and gamma-$^{32}$P ATP (New England Nuclear, Boston, MA). The approximately 975 bp fragment is specifically detected by the JR02 probe, strongly indicating that it comprises a portion of the cDNA for histidinol dehydrogenase.

The PCR fragment is excised from a low-gelling-temperature agarose gel, digested with EcoRI, and subcloned into the pBluescript plasmid (Stratagene, La Jolla, CA). The subcloned PCR insert is labeled with $^{32}$P by random priming using the PrimeTime kit (International Biotechnologies, New Haven, CT), and used to probe plaque lifts of the lambda ZAP II cabbage cDNA library described above. Positively-hybridizing plaques are purified and their inserts excised into the pBluescript plasmid in vivo using the method described by the manufacturer of the lambda ZAP II cloning vector (Stratagene, La Jolla, CA).

The DNA sequences of the plasmid cDNA inserts are determined by the dideoxy method using the Sequenase kit (United States Biochemical, Cleveland, OH). One plasmid subclone, designated pBSACabHdH7, is found to contain a 1613 bp insert that encodes a predicted protein that matches the sequences of the peptides derived from histidinol dehydrogenase, including the N-terminal peptide, indicating a full-length, or nearly full-length cDNA (see, SEQ ID NO: 1 for the DNA sequence).

EXAMPLE 13: **Preparation of Recombinant HDH using a Baculovirus Expression System**

**13.1** Insect cell culture

Sf9 cells were maintained as a monolayer culture in Grace's medium supplemented with 10 % (v/v) bovine fetal serum, 0.33 % (w/v) Yeastolate, 0.33 % (w/v) lactalbumin hydrolysate and 50 µg/ml of gentamycin sulfate at 27°C.

**13.2** Construction and isolation of recombinant virus

The cabbage histdinol dehydrogenase cDNA clone (pBSACabHdH7) was digested with KpnI and BamHI endonucleases. The KpnI-BamHI fragment (1.6 kb) of HDH, including the putative 93 bp chloroplast transit sequence coding region, was isolated and ligated with KpnI-BamHI double digested pVL1393. The recombinant transfer plasmid (pVL1339/HDH) containing the HDH cDNA was then integrated into the AcnNPV genome by cell-mediated homologous recombiantion. One µg of viral DNA was mixed with 80 µg of plasmid pVL1393/HDH DNA in 950 µl of HEPES buffer, 20 mM HEPES, 1 mM Na$_2$HPO$_4$, 5 mM KCl, 140 mM NaCl, and 10 mM glucose (pH 7.0). After precipitation with 50 µl of 2.5 M CaCl$_2$, the DNA segment was placed onto a monolayer culture of Sf9 cells and the culture was incubated at room temperature. After 1 h, the supernatant of the culture was replaced by 2 ml of culture medium. After incubation at 27°C for 4 days, the supernatant was diluted 10-1000-fold and subjected to plaque purification. The recombinant baculovirus AcNPV-HDH clones were initially identified by DNA hybridization analysis and later purified by visual screening under a dissecting microscope.

**13.3** Enzyme assay

Enzyme activity was spectrophotometrically measured by determining the absorbance of formed NADH at 340 nm as described previously (Nagai & Scheidegger, 1991). The enzyme reaction mixture contained 100 mM Gly-NaOH buffer (pH 9.2), 0.5 mM $MnCl_2$, 1 mM $NAD^+$, 2 mM histindinol, and enzyme. One unit of enzyme activity was defined as the amount of enzyme catalyzing the formation of 1 μm NADH per minute under the assay conditions.

**13.4** Expression and purification of recombinant HDH

The purified recombinant viral solution was amplified to a titer of 1-2 x $10^8$ PFU/ml. Cells were infected with virus at a MOI of 5-10 and incubateed at 27°C. 5 day post infected cells (1 x $10^8$ cells, 5 x 150 $cm^2$ mono-layered culture) were homogenized in 10 ml of 10 mM Tris-HCl buffer (pH 7.4) (buffer A) by using a Branson sonicator (SONIFIER 450). The cell homogenate was centrifuged at 10,000 g for 20 min, and the supernatant diluted with buffer A to an adquate concentration. The sample was desalted on Sephadex G-25. After incubation at 30°C for 2 h, the crude cell extract was applied onto a Mono-Q HR 16/10 anion-exchange column equilibrated with buffer A. After washing the column extensively with buffer A, the bound protein was eluted by a linear gradient of NaCl (0-300 mM) in buffer A. Active fractions were pooled and used for further studies.

As a result of the above enzyme purification two in its molecular weight differing proteins are obtained. The larger form, still containing 10 amino acids of the putative signal sequence, can be completely converted into the smaller form by incubation of the cell lysate for 2 h at 45°C. This proteolytic conversion is completely inhibited in the presence of the SH-protease inhibitor leupeptine but not PMSF [Phenylmethanesulfonyl fluoride]. The N-terminus of the major part [80% of the total] of the smaller form started at position -1, the minor part at +1 with regard to the N-terminus of the mature enzyme isolated from cabbage. All three forms of recombinant histidinol dehydrogenase showed identical kinetic properties with the wild-type cabbage enzyme.

EXAMPLE 14: **Preparation of cDNA clones encoding histidinol dehydrogenase from wheat**

Two wheat histidinol dehydrogenase cDNA clones [designated SA4 and SA5] are obtained analogous to the process described in EXAMPLE 12 using the cabbage clone as a hybridization probe under low-stringency conditions.

'In a first step total RNA is prepared from wheat germ [Sigma Chemical Co., St. Louis, MO] as described in EXAMPLE 12. Poly (A)+ RNA is isolated and a cDNA library is constructed as described in EXAMPLE 12. A portion of the library is grown as phage lambda plaques, and plaque filter lifts are prepared by standard methods as described in Maniatis *et al* (1982).

The cloned cabbage histidinol dehydrogenase cDNA isolated according to EXAMPLE 12 and shown in SEQ ID NO: 1 is radiolabeled as described in EXAMPLE 12 and hybridized to the filter lifts in a solution consisting of 7% sodium dodecyl sulfate, 0.5 M sodium phosphate pH 7, 1% bovine serum albumin, 1 mM EDTA [Church and Gilbert (1984)] at 50°C. The filters are washed in a solution consisting of 1% sodium dodecyl sulfate, 40 mM sodium phosphate pH 7, 125 mM sodium chloride, 1 mM EDTA at 50°C. These conditions of reduced hybridization and washing stringency allow detection of wheat cDNAs having substantial sequence homology to the cabbage sequence.

Positively hybridizing plaques are purified by sequential rounds of dilution, plating, and screening. Plasmids containing cDNA inserts from the positively-hybridizing phage are excised *in vivo* and their DNA sequences determined as described in EXAMPLE 12. The sequences of two closely related, but distinct wheat cDNAs, designated SA4 and SA5, are shown in SEQ ID NO: 2 and 3. Both sequences are 72% identical to the sequence of the cabbage clone shown in SEQ ID NO: 1, and are 96% identical to each other.

EXAMPLE 15: **Construction of pCGN1761 (a double CaMV 35S promoter/terminator cassette containing ampicillin resistance)**

pCGN1761 contains a double CaMV 35S promoter and the tml-3' region with an EcoRI site between contained in a pUC-derived plasmid backbone. The promoter-EcoRI-3' processing site cassette is bordered by multiple restriction sites for easy removal. The plasmid is derived by a series of steps (see below) from an initial double-35S plasmid, pCGN2113, which itself is derived from pCGN164, and pCGN638. The plasmid pCGN2113 is deposited with ATCC on March 22, 1989, accession number 40587 for the purposes of patent procedure in accordance with the regulations of the Budapest Treaty.

### 15.1 Construction of pCGN164

The AluI fragment of CaMV (bp 7144-7735) [Gardner *et al*, (1981)] is obtained by digestion with AluI and cloned into the HincII site of M13mp7 [Vieira and Messing (1982)] to create C614. An EcoRI digest of C614 produces the EcoRI fragment from C614 containing the 35S promoter which is cloned into the EcoRI site of pUC8 [Vieira and Messing (1982)] to produce pCGN146. To trim the promoter region, the BglII site (bp7670) is treated with BglII and Bal31 and subsequently a BglII linker is attached to the Bal31 treated DNA to produce pCGN147. pCGN147 is digested with EcoRI HphI and the resultant EcoRI-HphI fragment containing the 35S promoter is ligated into EcoRI-SmaII digested M13mp8 [Vieira and Messing, Gene 19: 259-268 (1982)] to create pCGN164.

### 15.2 Construction of pCGN638

Digestion of CaMV10 [Gardner *et al* (1981)] with BglII produces a BglII fragment containing a 35S promoter region (bp 6493-7670) which is ligated into the BamHI site of pUC19 [Norrander *et al* (1983)] to create pCGN638.

### 15.3 Construction of pCGN2113

pCGN164 is digested with EcoRV and BamHI to release a EcoRV-BamHI fragment which contained a portion of the 35S promoter (bp 7340-7433); pCGN638 is digested with HindIII and EcoRV to release a HindIII-EcoRV fragment containing a different portion of the 35S promoter (bp 6493-7340). These two fragments are ligated into pCGN986 which has been digested with HindIII and BamHI to remove the HindIII-BamHI fragment containing the 35S-promoter; this ligation produces pCGN639, which contains the backbone and tml-3' region from pCGN986 and the two 35S promoter fragments from pCGN164 and pCGN638. pCGN638 is digested with EcoRV and DdeI to release a fragment of the 35S promoter (bp 7070-7340); the fragment is treated with the Klenow fragment of DNA polymerase I to create blunt ends, and is ligated into the EcoRV site of pCGN639 to produce pCGN2113 having the fragment in the proper orientation.

### 15.4 Construction of pCGN1761

pCGN2113 is digested with EcoRI and the plasmid is ligated in the presence of a synthetic DNA adaptor containing an XbaI site and a BamHI site (the adaptor contains EcoRI sticky ends on either end, but the adjacent bases are such that an EcoRI site is not reconstructed at this location) to produce pCGN2113M. pCGN2113M is digested to completion with SacI and then subjected to partial digestion with BamHI. This DNA is then treated with T4 DNA polymerase to create blunt ends and an EcoRI linker is ligated into the blunt-ended plasmid. After transformation a plasmid clone which contains a unique EcoRI site between the promoter and the intact tml-3' region is selected and designated pCGN1761.

**BIBLIOGRAPHY**

**Adams,** J. Biol. Chem. 217:325-344 (1955)
**Allen** G, "Sequencing of proteins and peptides", in: Laboratory Techniques in Biochemistry and Molecular Biology, Vol 8, Eds. TS Work and RH Bordon, Elsevier, North-Holland, Biomedical Press, Amsterdam (1981)
**Bevan** *et al*, Nature 304: 184-187 (1983).
**Binding,** "Regeneration of Plants," in Plant Protoplasts, pp. 21-37 (CRC Press, Boca Raton, 1985)
**Bitar** *et al.*, Biochim. Biophys. Acta 493:429-440 (1977)
**Bradford,** Anal.Biochem. 72:248-254 (1976)
**Burger** *et al.*, Biochem. **J.** 181:771-774 (1979)
**Burger** *et al.*, Eur. J. Biochem. 116:137-142 (1981)
**Christou** *et al*, Plant Physiol. 87:671-674 (1988)
**Chu** *et al.*, Scientia Sinica, Vol. XVIII(No. 5):659-668 (1975)
**Crossway** *et al*, BioTechniques 4:320-334 (1986)
**Dale,** "Protoplast Culture and Plant Regeneration of Cereals and Other Recalcitrant Crops," in Protoplasts 1983 - Lecture Proceedings, pp. 31-41 (Birkhauser, Basel, 1983)
**Datta** *et al*, Bio/Technology 8:736-740 (1990)
**Davey**, "Recent Developments in the Culture and Regeneration of Plant Protoplasts," Protoplasts 1983)

**Davis** *et al*, <u>Advanced Bacterial Genetics</u>, Cold Spring Harbor Laboratory, Cold Spring Harbor, New York (1980)

**Donahue** *et al.*, Gene <u>18</u>:47-59 (1982)

**Dougall** and Fulton, Plant Physiol. <u>42</u>:941-945 (1967)

**Eccleston** *et al.*, J. Biol. Chem. <u>254</u>:11399-11404 (1979)

**Elzen** *et al.*, Plant Molecular Biology <u>5</u>: 299-392 (1985)

**Erlich,** (ed.), PCR Technology, Stockton Press, New York (1989)

**Evans** *et al*, "Protoplast Isolation and Culture," in <u>Handbook</u> <u>of Plant Cell Culture</u> <u>1</u>:124-176 (MacMillan Publishing Co., New York 1983)

**Fromm** *et al*, Bio/Technology <u>8</u>:833-839 (1990)

**Gardner** *et al*, Nucl. Acids Res. <u>9</u>: 2871-2888 (1981)

**Gordon-Kamm** *et al*, Plant Cell <u>2</u>:603-618 (1990)

**Grimsley** *et al*, EP-A 0 267 159

**Gorisch** and Holke, Eur. J. Biochem. <u>150</u>:305-308 (1985)

**Grubmeyer** *et al.*, Arch. Biochem. Biophys. <u>272</u>:311-317 (1989)

**Grubmeyer** *et al.*, Biochem. <u>28</u>:8174-8180 (1989)

**Hake** and Freeling, Nature <u>320</u>, 621-623 (1986)

**Heim** et al., Plant Physiol. <u>91</u>:1226-1231 (1989)

**Hinchee** *et al* Biotechnology <u>6</u>: 915-921 (1988)

**Hohn** *et al*, Current Topics in Microbiology and Immunology <u>96</u>:194-220 (1982)

**Hooykas-Van** Slogteren *et al.*, Nature <u>311</u>:763-764 (1984)

**Horsch** *et al*, Science <u>227</u>: 1229 (1985)

**Jefferson,** Plant Molecular Biology Reporter <u>5</u>: 387-405 (1987)

**Kanamori** *et al.*, J. Chromatog. <u>363</u>:231-242 (1986)

**Keesey** *et al.*, J. Biol. Chem. <u>254</u>:7427-7433 (1979)

**Klein** *et al*, Proc. Nat. Acad. Sci. USA <u>85</u>:4305-4309 (1988)

**Klein** *et al* Bio/Technology <u>6</u>:559-563 (1988)

**Klein** *et al*, Plant Physiol. <u>91</u>:440-444 (1989)

**Kumpaisal** *et al*, Plant Physiol. <u>85</u>:145-151 (1987)

**Laemmli,** Nature <u>227</u>:680-685 (1970)

**Lagrimini** *et al*, Proc. Natl. Acad. Sci. USA <u>84</u>, 7542-7546 (1987)

**Leung** *et al*, Technique <u>1</u>:11-15 (1989)

**McBride** *et al*, Plant Molecular Biology <u>14</u>: 266-276 (1990)

**Maniatis** *et al.*, <u>Molecular Cloning: A Laboratory Manual</u>, Cold Spring Harbor Laboratory, Cold Spring Harbor, New York (1982)

**Matsumoto** et al., J. Biochem. <u>85</u>:1091-1098 (1979)

**Maxam** and Gilbert, Proc. Nat. Acad. Sci. USA <u>74</u>:560-564 (1977)

**McCabe** *et al*, Bio/Technoiogy <u>6</u>:923-926 (1988)

**McCutcheon's** Detergents and Emulsifiers Annual," MC Publishing Corp. Ringwood, New Jersey, 1979

**Miller,** <u>Experiments in Molecular Genetics</u>, Cold Spring Harbor Laboratory, Cold Spring Harbor, New York (1972)

**Morelli** *et al*, Nature <u>315</u>: 200-204 (1985)

**Mullis** *et al*, Meth. Enzymol., <u>155</u>: 335-350 (1987)

**Nagai** et al, Proc. Nat. Acad. Sci. USA <u>88</u>:4133-4137 (1991)

**Negrutiu** et al., Mol. Gen. Genet. <u>199</u>:330-337 (1985)

**Neuhaus** *et al*, Theor Appl Genet <u>75</u>, 30-36 (1987)

**Norrander** *et al.*, Gene <u>26</u>: 101-106 (1983)

**Paszkowski** *et al*, EMBO J. <u>3</u>:2717-2722 (1984)

**Petit** *et al*, Mol. Gen. Genet. <u>202:</u> 388 (1986)

**Pierce** *et al*, <u>Plant Gene</u> <u>Systems and their Biology</u>, (Alan R. Liss, Inc.) pp. 301-310

**Poethig,** Nature <u>336</u>, 82-83 (1988)

**Reed** and Mann, Nucleic Acids Res. <u>13</u>:7207-7221 (1985)

**Riggs** *et al*, Proc. Nat. Acad. Sci. USA <u>83</u>:5602-5606 (1986)

**Saiki** *et al.*, Science <u>239</u>:487-491 (1988)

**Sanford** *et al*, U.S. Patent 4,945,050

**Sanford** *et al*, Particulate Science and Technology <u>5:27-37</u> (1987)

**Sanger,** Proc. Nat. Acad. Sci. USA <u>74</u>:5463-5467 (1977)]

**Schenk** and Hildebrandt, Can J Botany <u>50</u>: 199-204 (1972)

**Schocher** RJ *et al*, Bio/Technology, <u>4</u>: 1093-1096 (1986)

**Segel,** <u>Biochemical</u> <u>Calculations</u>, 2nd Edition, John Wiley and Sons, New York (1976); Suelter, <u>A Practical Guide</u>

to Enzymology, John Wiley and Sons, New York (1985).

**Shaffer** *et al.*, Brookhaven Symp. Biol. 23:250-270 (1972)

**Sherman** *et al*, Methods in Yeast Genetics, Cold Spring Harbor Laboratory, Cold Spring Harbor, New York (1983)

**Shillito** *et al*, Bio Technology, 3: 1099-1103 (1985)

**Shoutte** and Bolstein, Method. Enzymol. 100:457-468 (1983)

**Simpson** and Nice, Biochem Intl, 8: 787 (1984)

**Sisely** and Wood, "Encyclopedia of Surface Active Agents," Chemical Publishing Co., Inc. New York, 1980.

**Skyring** *et al.*, Anal. Biochem., 36:511-520 (1970)

**Somerville** and Ogren, In Edelman *et al* (eds) Methods in Chloroplast Molecular Biology, Elsevier Biomedical Press, pp. 129-138 (1982)

**Southern,** J. Mol. Biol. 98 :503 (1975)

**Strauss** *et al*, Planta 163:554-562 (1985)

**Strickler** *et al.*, Anal. Biochem. 140:553-566 (1984)

**Thompson** *et al*, EMBO J 6: 2519-2523 (1987)

**Tomes** *et al*, in Cereal Tissue and Cell Culture, pp. 175-203 (Bright, ed.) (1985)("Tomes 2")

**Velten** *et al.*, EMBO J. 3: 2723-2730 (1984)

**Vieira** and Messing, Gene 19: 259-268 (1982)

**Wang** Y-C *et al*, Plant Mol. Biol. 11: 433-439 (1988)

**Weissing** *et al*, Annual Rev. Genet. 22:421-477 (1988)

**Wiater** et al., Acta Biochim. Polonica 18:299-307 (1971)

**Wong** *et al.*, Phytochem. 20:1831-1834 (1981)

**Wong** and Mazelis, Phytochem. 20:1831-1834 (1981)

**Yamada** and Sato, Plant & Cell Physiol. 19:691-699 (1978)

**Yavada,** Mol. Gen. Genet. 170:109-111 (1979)

**Yourno** and Ino, J. Biol. Chem. 243:3273-3276 (1968)

**Zoller** and Smith, Meth. Enzymol. 100, 468-500 (1983)

**Patentliteratur:**

EP-A 0 189 707
EP-A 0 267 159
EP-A 0 306 139
EP-A 0 434 616
WO 89/07647
US-P 4,761.373

**SEQ ID NO: 1**
**SEQUENCE TYPE:** Nucleotide with corresponding protein
**SEQUENCE LENGTH:** 1613 base pairs

**STRANDEDNESS:** plus (coding) strand
**TOPOLOGY:** linear
**MOLECULE TYPE:** cDNA copy of mRNA

**ORIGINAL SOURCE**
**ORGANISM:** cabbage leaf tissue
**IMMEDIATE EXPERIMENTAL SOURCE**
**NAME OF CELL CLONE:** plasmid pBSACabHdH7

**PROPERTIES:** histidinol dehydrogenase activity

```
AATTCGGCAC  GAGCCTATTC GCCGGCGAAC ACACTCCTAT TTGAGCAAAC        50

CCGGTGAGG   ATG TCA TTC GAT CTA TCT CGT CTC TCC CTC ACT TCC   95
            Met Ser Phe Asp Leu Ser Arg Leu Ser Leu Thr Ser

TCG CCT CGT CTC TCG TTT CTC ACT CGC ACT GCT ACT AAG AAA GGA   140
Ser Pro Arg Leu Ser Phe Leu Thr Arg Thr Ala Thr Lys Lys Gly

TTC GTT AGG TGT TCG ATG AAG TCG TAC AGA TTA TCT GAA CTT AGT   185
Phe Val Arg Cys Ser Met Lys Ser Tyr Arg Leu Ser Glu Leu Ser

TTC TCT CAA GTT GAG AAC TTG AAG GCA CGC CCT CGC ATT GAC TTC   230
Phe Ser Gln Val Glu Asn Leu Lys Ala Arg Pro Arg Ile Asp Phe

TCT TCC ATT TTC ACC ACT GTT AAC CCA ATC ATC GAC GCT GTT CGT   275
Ser Ser Ile Phe Thr Thr Val Asn Pro Ile Ile Asp Ala Val Arg

AGC AAA GGA GAT ACT GCT GTC AAA GAG TAT ACA GAG AGA TTT GAC   320
Ser Lys Gly Asp Thr Ala Val Lys Glu Tyr Thr Glu Arg Phe Asp

AAA GTC CAG CTC AAT AAA GTG GTG GAG GAT GTG TCC GAA CTT GAT   365
Lys Val Gln Leu Asn Lys Val Val Glu Asp Val Ser Glu Leu Asp

ATC CCT GAG CTC GAC TCC GCA GTT AAA GAA GCG TTT GAT GTT GCG   410
Ile Pro Glu Leu Asp Ser Ala Val Lys Glu Ala Phe Asp Val Ala
```

```
TAT GAC AAC ATT TAT GCA TTT CAC TTT GCC CAA ATG TCA ACT GAG 455
Tyr Asp Asn Ile Tyr Ala Phe His Phe Ala Gln Met Ser Thr Glu

AAA AGC GTT GAG AAT ATG AAA GGT GTA AGA TGT AAA AGG GTG TCG 500
Lys Ser Val Glu Asn Met Lys Gly Val Arg Cys Lys Arg Val Ser

AGA TCT ATT GGT TCT GTT GGT CTT TAT GTG CCT GGT GGA ACT GCT 545
Arg Ser Ile Gly Ser Val Gly Leu Tyr Val Pro Gly Gly Thr Ala

GTT TTG CCA TCT ACT GCT TTG ATG CTT GCT ATT CCT GCT CAG ATT 590
Val Leu Pro Ser Thr Ala Leu Met Leu Ala Ile Pro Ala Gln Ile

GCT GGA TGT AAA ACA GTT GTC CTC GCA ACT CCA CCA ACT AAG GAA 635
Ala Gly Cys Lys Thr Val Val Leu Ala Thr Pro Pro Thr Lys Glu

GGA AGC ATA TGT AAG GAG GTG CTG TAT TGT GCC AAG AGG GCT GGT 680
Gly Ser Ile Cys Lys Glu Val Leu Tyr Cys Ala Lys Arg Ala Gly

GTA ACT CAC ATT CTT AAA GCT GGT GGA GCA CAG GCT ATA GCT GCC 725
Val Thr His Ile Leu Lys Ala Gly Gly Ala Gln Ala Ile Ala Ala

ATG GCC TGG GGG ACA GAT TCT TGT CCT AAG GTT GAG AAG ATT TTT 770
Met Ala Trp Gly Thr Asp Ser Cys Pro Lys Val Glu Lys Ile Phe

GGT CCT GGG AAT CAG TAT GTT ACA GCT GCT AAG ATG ATT CTG CAA 815
Gly Pro Gly Asn Gln Tyr Val Thr Ala Ala Lys Met Ile Leu Gln

AAC AGC GAG GCA ATG GTT TCG ATT GAT ATG CCT GCT GGC CCT TCA 860
Asn Ser Glu Ala Met Val Ser Ile Asp Met Pro Ala Gly Pro Ser

GAA GTT CTT GTT ATC GCT GAT GAA CAT GCT AGT CCA GTT TAC ATT 905
Glu Val Leu Val Ile Ala Asp Glu His Ala Ser Pro Val Tyr Ile

GCA GCC GAC TTA CTT TCT CAG GCT GAG CAT GGT CCA GAT AGT CAG 950
Ala Ala Asp Leu Leu Ser Gln Ala Glu His Gly Pro Asp Ser Gln

GTT GTT CTT GTT GTT GTA GGC GAT GGT GTA AAT CTC AAA GCC ATC 995
Val Val Leu Val Val Val Gly Asp Gly Val Asn Leu Lys Ala Ile

GAA GAA GAA ATT GCT AAA CAA TGC AAA AGC CTT CCT AGA GGC GAG 1040
Glu Glu Glu Ile Ala Lys Gln Cys Lys Ser Leu Pro Arg Gly Glu

TTT GCT TCA AAA GCT CTA AGT CAC AGC TTC ACA GTA TTT GCT CGA 1085
Phe Ala Ser Lys Ala Leu Ser His Ser Phe Thr Val Phe Ala Arg

GAT ATG ATT GAG GCA ATA ACT TTC TCA AAC CTG TAT GCA CCT GAA 1130
Asp Met Ile Glu Ala Ile Thr Phe Ser Asn Leu Tyr Ala Pro Glu

CAC TTG ATC ATC AAT GTC AAA GAC GCT GAG AAA TGG GAG GGA CTG 1175
His Leu Ile Ile Asn Val Lys Asp Ala Glu Lys Trp Glu Gly Leu

ATA GAG AAC GCA GGT TCG GTT TTC ATA GGG CCG TGG ACT CCT GAG 1220
Ile Glu Asn Ala Gly Ser Val Phe Ile Gly Pro Trp Thr Pro Glu
```

```
AGT GTT GGT GAT TAC GCG AGC GGG ACA AAC CAC GTT CTT CCA ACG 1265
Ser Val Gly Asp Tyr Ala Ser Gly Thr Asn His Val Leu Pro Thr

TAC GGG TAT GCG AGA ATG TAC AGT GGC GTC TCT CTC GAC TCT TTC 1310
Tyr Gly Tyr Ala Arg Met Tyr Ser Gly Val Ser Leu Asp Ser Phe

CTG AAG TTC ATG ACT GTA CAG TCC TTG ACA GAG GAA GGT CTG CGA 1355
Leu Lys Phe Met Thr Val Gln Ser Leu Thr Glu Glu Gly Leu Arg

AAC CTT GGT CCG TAT GTT GCG ACT ATG GCT GAA ATT GAA GGT CTA 1400
Asn Leu Gly Pro Tyr Val Ala Thr Met Ala Glu Ile Glu Gly Leu

GAT GCA CAC AAG AGA GCC GTC ACT CTC AGA CTC AAG GAT ATC GAA 1445
Asp Ala His Lys Arg Ala Val Thr Leu Arg Leu Lys Asp Ile Glu

GCC AAA CAG ACC CAA ACA AAG TGA      AATGAATGGC   TGTCTTCATA 1489
Ala Lys Gln Thr Gln Thr Lys *

TTCAAATGAG   GTGACATTGG   TTTAAAGAGA   TAATAATAAT   AAGAATAATA 1539

TAAGAGATGT   TGTAACACTC   TCTGTCATAA   TCTGGATTTT   CTTTCATTAT 1589

TGAAATTTGA   ATCCTTTCAC   GCGT                                1613
```

**SEQ ID NO:** 2
**SEQUENCE TYPE:** Nucleotide
**SEQUENCE LENGTH:** 1137 base pairs

**STRANDEDNESS:** plus (coding) strand
**TOPOLOGY:** linear
**MOLECULE TYPE:** cDNA copy of mRNA

**ORIGINAL SOURCE**
**ORGANISM:** wheat
**IMMEDIATE EXPERIMENTAL SOURCE**
**NAME OF CELL CLONE:** plasmid

**PROPERTIES:** portion of histidinol dehydrogenase mRNA
**FEATURES:** stop codon at 951

```
GGCACGAGGC TGTATGTTCC AGGGGGCACT GCAGTCTTGC CTTCAACTGC        50

ATTGATGCTT GCTGTGCCTG CACAGATTGC TGGATGCAAA ACTGTAGTTC       100

TTGCCACACC CCCTAGTCGT GATGGTAGCA TATGTAAGGA GGTTCTTTAC       150

TGTGCTAAAA AAGCTGGTGT TACACACATA CTAAAAGCTG GGGGAGCTCA       200

GGCAATCTCC GCTATGGCAT GGGGAACTGC ATCATGCCCA AAGGTTGAGA       250

AAATATTTGG TCCCGGCAAC CAGTATGTCA CAGCTGCTAA AATGATTCTT       300

CAGAACAGCG AAGCTATGGT CTCTATAGAC ATGCCTGCTG GCCCTTCAGA       350

AGTTCTGGTA ATCGCTGACA AATATGCCAA CCCAGTTCAT GTTGCCGCTG       400

ATCTGCTATC TCAGGCAGAA CATGGCCCAG ATAGTCAGGT TGTTCTAGTT       450

ATTGCGGGAG ATGGTGTTGA TTTAGCTGCC ACTGAAGCAG AAGTTAGCAA       500

GCAGTGTGAT GCTCTTCCTA GAGGCGACTT TGCTTCCAAA GCACTTGGCC       550


ACAGTTTCAC TGTGTTTGCC AGAGATATGG CTGAGGCATT ATCGTTCTCA       600

AATATGTATG CTCCTGAGCA TTTGATCATC AATGTAAAGG ATGCTGAGCA       650

ATGGGAGGAG CTCATCGAGA ATGCAGGGTC AGTTTTCTTG GGACAATGGA       700

CCCCCGAGAG CGTCGGTGAC TACGCTAGTG GGACGAACCA CGTCCTCCCA       750

ACCTACGGTT ACGCGAGGAT GTACAGCGGT GTGTCGCTGA ATTCTTTCCT       800

CAAGTACATC ACTGTTCAAT CCCTGACTGA AGNAGGGCTG AGAAGGCTGG       850

GTCCCTACGT CGCAAAGATG GCAGAAGTCG AAGGCCTAGA AGCGCACAAG       900

CGAGCTGTTA CCCTCAGACT GCAAGAGATC GAAGCCACTG TAACTGTTTA       950

AACTGTTAAC ATGGTCTGTT GTGCCAGTCT ATTTGATGCT TGGGAACTGA      1000

TATTTTTCTT AATTCTTATC GGATGGGACT TGCCAGAGTT TAAGGTGAAT      1050

ACGAAGCTGT TTCATGGAAT AATGCTACTT ATGTCGATCC GTTAAATGAA      1100

TAAACTTGTG AGTACAGTCT CTAAAAAAAA AAAAAAA                    1137
```

**SEQ ID NO:** 3
**SEQUENCE TYPE:** Nucleotide
**SEQUENCE LENGTH:** 995 base pairs

**STRANDEDNESS:** plus (coding) strand
**TOPOLOGY:** linear
**MOLECULE TYPE:** cDNA copy of mRNA

**ORIGINAL SOURCE**
**ORGANISM:** wheat
**IMMEDIATE EXPERIMENTAL SOURCE**
**NAME OF CELL CLONE:** plasmid

**PROPERTIES:** portion of histidinol dehydrogenase mRNA
**FEATURES:** stop codon at 828

```
GGCACGAGAT  GTAAGGAGGT  TCTTTACTGT  GCCAAAAAAG  CTGGTGTTAC      50

ACACATACTA  AAAGCTGGGG  GAGCTCAGGC  AATCTCCGCT  ATGGCATGGG     100

GAACCGCATC  ATGCCCAAAG  GTTGAGAAAA  TATTTGGTCC  TGGCAACCAG     150

TATGTCACAG  CTGCTAAAAT  GATTCTTCAG  AACAGCGAAG  CTATGGTCTC     200

TATAGACATG  CCTGCTGGCC  CTTCAGAAGT  TCTGGTAATT  GCTGACAAAT     250

ATGCCAACCC  AGTTCATGTT  GCCGCTGATC  TGCTATCTCA  GGCAGAACAT     300

GGCCCAGATA  GTCAGGTTGT  TCTAGTTATT  GCTGGAGATG  GTGTTGATTT     350

AGGTGCCATT  GAAGCAGAAG  TTAGCAAGCA  GTGCGATGCT  CTTCCTAGAG     400

GCGACTTTGC  TTCCAAAGCA  CTTGGCCACA  GTTTCACTGT  GTTTGNCAGA     450

GATATGGTTG  AGGNATTATC  GTTCTCAAAT  ATGTATGCTC  CTGAGCATTT     500

GATCATCAAC  GTAAAGGATG  CTGAGCAATG  GGAGGAGCTC  ATCGAGAACG     550


CAGGGTCAGT  TTTCTTGGGA  CAATGGACCC  CCGAGAGCGT  GGGTGACTAT     600

GCCAGTGGGA  CGAACCACGT  CCTCCCAACC  TACGGTTACG  CGAGGATGTA     650

CAGCGGTGTG  TCGCTGAACT  CTTTCCTCAA  GTACATCACT  GTTCAATCCC     700

TGACTGAAGA  AGGGCTGAGA  AGGCTGGGCC  CCTACGTCGC  AAAGATGGCA     750

GAAGTCGAAG  GGCTAGAAGC  GCACAAGCGA  GCTGTTACCC  TCAGACTGCA     800

AGAGGTCGAA  GCCACTGTAA  CTGTGTAAAC  TGTTAGCATG  GTCTGTTGTG     850

CCAGTCTATT  TGATGCTTGG  GGACTGATAT  TTTTCTTCAT  TTTTATCGGA     900

TGGAACTTGC  CAGAGTTTAA  GGTGAAGAAT  ACGAAGCTGT  TTCATGGAAT     950

AATGCTACTT  ATGTCGATCT  GTTAAAAAAA  AAAAAAAAAA  AAAAA          995
```

**Claims**

1. A plant protein having an amino acid sequence identical or homologous to at least 70 percent of a protein sequence of SEQ ID NO: 1.

2. A protein according to claim 1 wherein said amino acid sequence is homologous to at least 80 percent of said protein sequence of SEQ ID NO: 1.

3. A protein according to claim 2 wherein said amino acid sequence is homologous to at least 90 percent of said protein sequence of SEO ID NO: 1.

4. A protein according to claim 3 wherein said amino acid sequence is homologous to at least 99 percent of said protein sequence of SEQ ID NO: 1.

5. A DNA sequence identical or homologous to at least 60 percent of the DNA sequence of SEQ ID NO: 1.

6. A DNA sequence according to claim 5, wherein said DNA sequence is homologous to at least 75 percent of the DNA sequence of SEQ ID NO: 1.

7. A DNA sequence according to claim 6, wherein said DNA sequence is homologous to at least 90 percent of the DNA sequence of SEQ ID NO: 1.

8. A DNA sequence according to claim 6, wherein said DNA sequence is the DNA sequence of SEQ ID NO: 1.

9. A purified recombinant plant protein expressed from a DNA sequence according to any one of claims 5 to 8.

10. A method of producing a DNA sequence coding for a plant protein exhibiting histidinol dehydrogenase activity as claimed in any one of claims 5 to 8, which method comprises:

   (a) preparing a genomic or a cDNA library from a suitable source organism using an appropriate cloning vector;
   (b) hybridizing the library with a probe molecule;
   (c) identifying positive hybridizations of the probe to the DNA clones from the library that is clones potentially containing the nucleotide sequence corresponding to the amino acid sequence for histidinol dehydrogenase.

11. A method of producing a DNA sequence coding for a plant protein exhibiting histidinol dehydrogenase activity as claimed in any one of claims 5 to 8, which method comprises:

   (a) preparing total DNA from a genomic or a cDNA library;
   (b) using the DNA of step (a) as a template for PCR reaction with primers representing low degeneracy portions of the amino acid sequence of histidinol dehydrogenase.

12. An assay to identify inhibitors of a protein exhibiting histidinol dehydrogenase activity comprising:

   (a) incubating a first sample of a protein exhibiting histidinol dehydrogenase activity according to claims 1-4 and its substrate;
   (b) measuring an uninhibited reactivity of the histidinol dehydrogenase from step (a);
   (c) incubating a first sample of histidinol dehydrogenase and its substrate in the presence of a second sample comprising an inhibitor compound;
   (d) measuring an inhibited reactivity of the histidinol dehydrogenase from step (c); and
   (e) comparing the reactivity of the inhibited histidinol dehydrogenase to the reactivity of the uninhibited enzyme to identify an inhibitor molecule capable of decreasing histidinol dehydrogenase activity.

13. An assay to identify inhibitor-resistant mutants of a protein exhibiting histidinol dehydrogenase activity comprising:

   (a) incubating a first sample of a protein exhibiting histidinol dehydrogenase activity according to claims 1-4 having wild-type enzyme activity and its substrate in the presence of a second sample comprising a histidinol dehydrogenase inhibitor;
   (b) measuring an unmutated reactivity of the histidinol dehydrogenase from step (a);
   (c) incubating a first sample of a mutated histidinol dehydrogenase and its substrate in the presence of a second sample comprising a histidinol dehydrogenase inhibitor;
   (d) measuring a mutated reactivity of the mutated histidinol dehydrogenase from step (c); and
   (e) comparing the reactivity of the mutant histidinol dehydrogenase to the reactivity of the wild-type enzyme to identify a mutant histidinol dehydrogenase with increased reactivity compared to the wild-type enzyme.

14. Use of the DNA sequence according to any one of claims 5 to 8 or of fragments thereof as a hybridization probe in a process of identifying further DNA sequences of homologous or heterologous source origin encoding a protein exhibiting histidinol dehydrogenase activity.

15. Use of the DNA sequence according to any one of claims 5 to 8 or of fragments thereof as a RFLP marker.

**Patentansprüche**

1. Pflanzenprotein, das eine Aminosäure-Sequenz aufweist, die identisch mit oder homolog zu mindestens 70 % einer Protein-Sequenz SEQ ID Nr. 1 ist.

2. Protein nach Anspruch 1, worin die genannte Aminosäure-Sequenz homolog zu mindestens 80 % der genannten Protein-Sequenz SEQ ID Nr. 1 ist.

3. Protein nach Anspruch 2, worin die genannte Aminosäure-Sequenz homolog zu mindestens 90 % der genannten Protein-Sequenz SEQ ID Nr. 1 ist.

4. Protein nach Anspruch 3, worin die genannte Aminosäure-Sequenz homolog zu mindestens 99 % der genannten Protein-Sequenz SEQ ID Nr. 1 ist.

5. DNA-Sequenz, die identisch mit oder homolog zu mindestens 60 % der DNA-Sequenz SEQ ID Nr. 1 ist.

6. DNA-Sequenz nach Anspruch 5, worin die genannte DNA-Sequenz homolog zu mindestens 75 % der DNA-Sequenz SEQ ID Nr. 1 ist.

7. DNA-Sequenz nach Anspruch 6, worin die genannte DNA-Sequenz homolog zu mindestens 90 % der DNA-Sequenz SEQ ID Nr. 1 ist.

8. DNA-Sequenz nach Anspruch 6, worin die genannte DNA-Sequenz die DNA-Sequenz SEQ ID Nr. 1 ist.

9. Gereinigtes rekombinantes Pflanzenprotein, das von einer DNA-Sequenz nach einem der Ansprüche 5 bis 8 exprimiert worden ist.

10. Verfahren zur Herstellung einer DNA-Sequenz, die ein eine Histidinol-Dehydrogenase-Aktivität aufweisendes Pflanzenprotein nach einem der Ansprüche 5 bis 8 codiert, wobei das Verfahren umfasst:

   (a) die Herstellung einer Genom- oder cDNA-Genbibliothek aus einem geeigneten Quellen-Organismus unter Verwendung eines geeigneten Klonierungsvektors;
   (b) das Hybridisieren der Genbibliothek mit einem Sondenmolekül;
   (c) das Identifizieren von positiven Hybridisierungen der Sonde mit den DNA-Klonen aus der Genbibliothek, d.h. mit Klonen, die potentiell die Nucleotid-Sequenz enthalten, die der Aminosäure-Sequenz für die Histidinol-Dehydrogenase entspricht.

11. Verfahren zur Herstellung einer DNA-Sequenz, die ein eine Histidinol-Dehydrogenase-Aktivität aufweisendes Pflanzenprotein nach einem der Ansprüche 5 bis 8 codiert, wobei das Verfahren umfasst:

   (a) die Herstellung der Gesamt-DNA aus einer Genom- oder cDNA-Genbibliothek;
   (b) die Verwendung der DNA der Stufe (a) als Matrize für die PCR-Reaktion mit Startern, welche die Abschnitte der Aminosäure-Sequenz von Histidinol-Dehydrogenase mit geringer Entartung darstellen.

12. Assay zur Identifizierung von Inhibitoren eines Proteins, das eine Histidinol-Dehydrogenase-Aktivität aufweist, der umfasst

   (a) das Inkubieren einer ersten Probe eines eine Histidinol-Dehydrogenase-Aktivität aufweisenden Proteins nach den Ansprüchen 1 bis 4 und ihres Substrats;
   (b) die Bestimmung der nicht-inhibierten Reaktionsfähigkeit der Histidinol-Dehydrogenase aus der Stufe (a);
   (c) das Inkubieren einer ersten Probe der Histidinol-Dehydrogenase und ihres Substrats in Gegenwart einer zweiten Probe, die eine Inhibitor-Verbindung umfasst;
(d) das Bestimmen der inhibierten Reaktionsfähigkeit der Histidinol-Dehydrogenase aus der Stufe (c); und
(e) das Vergleichen der Reaktionsfähigkeit der inhibierten Histidinol-Dehydrogenase mit der Reaktionsfähigkeit des nicht-inhibierten Enzyms, um ein Inhibitor-Molekül zu identifizieren, das die Histidinol-Dehydrogenase-Aktivität verringern kann.

13. Assay zur Identifizierung von Inhibitor-resistenten Mutanten eines eine Histidinol-Dehydrogenase-Aktivität aufweisenden Proteins, der umfasst:

(a) das Inkubieren einer ersten Probe eines eine Histidinol-Dehydrogenase-Aktivität aufweisenden Proteins nach den Ansprüchen 1 bis 4, das eine Enzym-Aktivität vom Wild-Typ aufweist, und ihres Substrats in Gegenwart einer zweiten Probe, die einen Histidinol-Dehydrogenase-Inhibitor umfasst;
(b) das Bestimmen der nicht-mutierten Reaktionsfähigkeit der Histidinol-Dehydrogenase aus der Stufe (a);
(c) das Inkubieren einer ersten Probe einer mutierten Histidinol-Dehydrogenase und ihres Substrats in Gegenwart einer zweiten Probe, die einen Histidinol-Dehydrogenase-Inhibitor umfasst;
(d) die Bestimmung der mutierten Reaktionsfähigkeit der mutierten Histidinol-Dehydrogenase aus der Stufe (c); und
(e) das Vergleichen der Reaktionsfähigkeit der mutierten Histidinol-Dehydrogenase mit der Reaktionsfähigkeit des Enzyms vom Wild-Typ, um eine Mutanten-Histidinol-Dehydrogenase mit einer erhöhten Reaktionsfähigkeit, verglichen mit dem Enzym vom Wild-Typ, zu identifizieren.

14. Verwendung der DNA-Sequenz nach einem der Ansprüche 5 bis 8 oder von Fragmenten davon als Hybridisierungs-Sonde in einem Verfahren zur Identifizierung weiterer DNA-Sequenzen eines homologen oder heterologen Quellenursprungs, die ein eine Histidinol-Dehydrogenase-Aktivität inhibierendes Protein codieren.

15. Verwendung der DNA-Sequenz nach einem der Ansprüche 5 bis 8 oder von Fragmenten davon als RFLP-Marker.


**Revendications**

1. Protéine végétale ayant une séquence d'acides aminés identique ou homologue à au moins 70 pour cent d'une séquence protéïque de SEQ ID NO: 1

2. Protéine selon la revendication 1, dans laquelle ladite séquence d'acides aminés est homologue à au moins 80 pour cent de ladite séquence de protéine de SEQ ID NO: 1.

3. Protéine selon la revendication 2, dans laquelle ladite séquence d'acides aminés est homologue à au moins 90 pour cent de ladite séquence de protéine de SEQ ID NO: 1

4. Protéine selon la revendication 3, dans laquelle ladite séquence d'acides aminés est homologue à au moins 99 pour cent de ladite séquence de protéine de SEQ ID NO: 1.

5. Séquence d'ADN identique ou homologue à au moins 60 pour cent de la séquence d'ADN de SEQ ID NO: 1

6. Séquence d'ADN selon la revendication 5, dans laquelle ladite séquence d'ADN est homologue à au moins 75 pour cent de la séquence d'ADN de SEQ ID NO: 1.

7. Séquence d'ADN selon la revendication 6, dans laquelle ladite séquence d'ADN est homologue à au moins 90 pour cent de la séquence d'ADN de SEQ ID NO: 1.

8. Séquence d'ADN selon la revendication 6, dans laquelle ladite séquence d'ADN est la séquence d'ADN de SEQ ID NO:1.

9. Protéine végétale recombinante purifiée exprimée à partir d'une séquence d'ADN selon l'une quelconque des revendications 5 à 8.

10. Procédé de production d'une séquence d'ADN codant une protéine végétale ayant l'activité histidinol déshydrogénase telle que celle revendiquée dans l'une quelconque des revendications 5 à 8, ledit procédé comprenant :

(a) la préparation d'une bibliothèque génomique ou d'ADNc à partir d'un organisme source approprié en utilisant un vecteur de clonage approprié ;

(b) l'hybridation de la génothèque avec une molécule sonde ;

(c) l'identification des hybridations positives des clones d'ADN de la génothèque c'est à dire des clones pouvant contenir la séquence nucléotidique correspondant à la séquence d'acides aminés de l'histidinol déshydrogénase.

11. Méthode de production d'une séquence d'ADN codant une protéine végétale ayant l'activité histidinol déshydrogénase telle que celle revendiquée dans l'une quelconque des revendications 5 à 8, ladite méthode comprend :

(a) la préparation de l'(ADN total à partir d'une génothèque génomique ou d'ADNc

(b) l'utilisation de l'ADN de l'étape (a) comme matrice pour la réaction de PCR avec des amorces représentant des portions de faible dégénérescence de la séquence d'acides aminés de l'histidinol déshydrogénase.

12. Essai d'identification des inhibiteurs d'une protéine ayant l'activité histidinol déshydrogénase comprenant :

(a) l'incubation d'un premier échantillon d'une protéine ayant l'activité histidinol déshydrogénase selon les revendications 1-4 et de son substrat ;

(b) la mesure de la réactivité non inhibée de l'histidinol déshydrogénase de l'étape (a) ;

(c) l'incubation d'un premier échantillon de l'histidinol déshydrogénase et de son substrat en présence d'un second échantillon comprenant un composé inhibiteur ;

(d) la mesure de la réactivité non inhibée de l'histidinol déshydrogénase de l'étape (c) ; et

(e) la comparaison de la réactivité de l'histidinol déshydrogénase inhibée avec la réactivité de l'enzyme non inhibé pour identifier une molécule inhibitrice capable de diminuer l'activité histidinol déshydrogénase.

13. Essai pour identifier des mutants résistants à l'inhibiteur d'une protéine ayant l'activité histidinol déshydrogénase comprenant :

(a) l'incubation d'un premier échantillon d'un protéine ayant l'activité histidinol déshydrogénase selon les revendications 1-4 ayant une activité enzymatique de type sauvage, et de son substrat en présence d'un second échantillon comprenant un inhibiteur de l'histidinol déshydrogénase ;

(b) la mesure d'une réactivité non mutée de l'histidinol déshydrogénase de l'étape (a) ;

(c) l'incubation d'un premier échantillon d'une histidinol déshydrogénase mutée et de son substrat en présence d'un second échantillon comprenant un inhibiteur de l'histidinol déshydrogénase ;

(d) la mesure d'une réactivité mutée de l'histidinol déshydrogénase mutée de l'étape ( c) ; et

(e) la comparaison de la réactivité de l'histidinol déshydrogénase mutante à la réactivité de l'enzyme de type sauvage pour identifier une histidinol déshydrogénase mutante ayant une réactivité augmentée par rapport à l'enzyme de type sauvage.

14. Utilisation de la séquence d'ADN selon l'une quelconque des revendications 5 à 8 ou de fragments de celle-ci comme sonde d'hybridation dans un procédé d'identification d'autres séquences d'ADN d'origine source homologue ou hétérologue codant une protéine ayant l'activité l'histidinol déshydrogénase.

15. Utilisation de la séquence d'ADN selon l'une quelconque des revendications 5 à 8 ou de fragments de celle-ci comme marqueur de RFLP.

Fig. 1 HISTIDINOL-SEPHAROSE-4B AFFINITY CHROMATOGRAPHY

# Fig. 2   DEPENDENCY OF HISTIDINOL DEHYDROGENASE ACTIVITY ON pH